# EUROPEAN PATENT APPLICATION

(11) **EP 2 298 302 A1**
(43) Date of publication of application: **23.03.2011**
(21) Application number: 10010605.3
(22) Date of filing: 10.06.2003
(51) Int. Cl.: A61K 31/485, A61K 9/70, A61K 31/445, B09B 3/00, A61F 13/00

(54) **Disposal systems of transdermal delivery devices to prevent misuse of the active agents contained therein**

(30) Priority: 10.06.2002 US 387800 P
(62) Divisional of application: 07003694.2
(71) Applicant: EURO-CELTIQUE S.A., 1653 Luxembourg (LU)
(72) Inventor: Baker, Carl J., Middletown, NY 10941-5004 (US); Shevchuk, Ihor, Yonkers, NY 10710 (US)
(74) Representative: Maiwald, Walter

(57) **Abstract**

The present invention relates to a transdermal delivery device disposal system for disposing of a transdermal delivery device containing at least one pharmaceutically active component. The disposal system contains at least one sheet or substrate on which an adhesive is coated; and one or more of the following components: (a) monomer, and optionally comonomer(s), to be polymerized; (b) an initiator, and optionally co-initiator(s), to initiate the polymerization; (c) at least one crosslinking agent to crosslink the (co)polymer; and (d) at least one deactivating agent to chemically alter, to degrade, and/or to deactivate the active component(s) contained in the transdermal delivery device, such as an opioid antagonist or an opioid agonist deactivating agent so that the euphoric effects of the active component(s) contained in the transdermal delivery device are at least temporarily inhibited, diminished, or halted *in vivo*, wherein at least one adhesive-coated sheet or substrate adheres to, immobilizes, or isolates, and prevents, inhibits, or diminishes the misuse or abuse of the active component contained in, at least one transdermal delivery device.

## Description

### FIELD OF THE INVENTION

The present invention relates to a disposal system for preventing, inhibiting, and/or diminishing the intentional and/or inadvertent misuse or abuse of a transdermal delivery device ("TDD") containing an active pharmaceutical agent, such as an opioid. The present invention also relates to a method for disposing of a TDD and/or for preventing the misuse or abuse of a TDD or any ingredient of a TDD.

### BACKGROUND OF THE INVENTION

Transdermal dosage forms are convenient dosage forms for delivering many different active therapeutically effective agents, including but not limited to analgesics, such as opioid analgesics. Typical opioid analgesics include, but are not limited to, fentanyl, buprenorphine, etorphines, and other high potency narcotics. Other therapeutically effective agents which may be delivered by a transdermal delivery system include, but are not limited to, anti-emetics (scopolamine), cardiovascular agents (nitrates and clonidine), hormones (estrogen and testosterone), nicotine, vitamins, dietary supplements, etc.

The most common transdermal dosage form is a diffusion-driven transdermal system (transdermal patch) using either a fluid reservoir or a drug-in-adhesive matrix system. Other transdermal dosage forms include, but are not limited to, topical gels, lotions, ointments, transmucosal systems and devices, and iontophoretic (electrical diffusion) delivery systems.

Transdermal dosage forms are particularly useful for timed-release and sustained-release of active agents. However, many dosage forms, and particularly those for timed and sustained release of active agent(s), contain large amounts of active agent(s), often many times the actual absorbed dose. Often, the dosage form contains an excess of active agent or delivers less than the entire amount of its active agent to the subject being treated. This results in most of the active agent remaining in the dosage form after use. Both the unused dosage form and the portion of active agent that remains in the dosage form after use are subject to potential illicit abuse, particularly if the active agent is a narcotic or a controlled substance. For example, used dosage forms containing excess or unused opioids may be tampered with by chewing or extraction by a drug abuser. Even careful disposal of used dosage forms may not be completely effective in preventing abuse, particularly in cases of incomplete or partial compliance.

U.S. Patent No. 5,804,215 to Cubbage et al. relates to a disposal system for a transdermal patch containing a medicament, such as nicotine, comprising an adhesive coated flexible tear-resistant substrate. The used transdermal patch is adhered to the substrate in order to encapsulate and prevent access to the transdermal patch. Rubber based adhesives are preferred by Cubbage

U.S. Patents No. 5,149,538 to Granger et al. and 5,236,714 to Lee et al. each relate to misuse-resistive dosage forms for the transdermal delivery of opioids. In these patents, the respective transdermal patches are provided with an opioid antagonist which is intended to be extracted from the patch if the patch is chewed or subjected to an extraction process. The opioid antagonist is intended to block any euphoric effects sought through abuse of the dosage form.

The inadvertent and/or intentional misuse and/or abuse of transdermal delivery devices remains a significant health problem. Thus, there is a need for a transdermal delivery disposal system that is less susceptible to abuse than is presently known in the art.

### SUMMARY OF THE INVENTION

One embodiment of the invention relates to a TDD disposal system comprising at least a first substrate having an adhesive coating on one face thereof; and one or more of the following components present at least in or on the adhesive coating or in or on the first substrate, or any combination thereof:
(a) a monomer, and optionally at least one comonomer;
(b) an initiator, and optionally at least one co-initiator;
(c) at least one crosslinking agent; and
(d) at least one active agent deactivating agent, *e.g*., an opioid antagonist for use with a TDD comprising an opioid agonist.

Another embodiment of the invention relates to a TDD disposal system further comprising one or more of the following components present at least in or on the adhesive coating or in or on a second substrate, or any combination thereof:
(a) a monomer, and optionally at least one comonomer;
(b) an initiator, an optionally at least one co-initiator;
(c) at least one crosslinking agent; and
(d) at least one active agent deactivating agent.

An additional embodiment of the invention relates to a kit comprising a transdermal delivery device comprising at least one active agent; and a TDD disposal system; where the TDD disposal system comprises:
(i) at least a first substrate having an adhesive coating on one face thereof; and
(ii) one or more of the following components present at least in or on the adhesive coating or in or on the first substrate, or any combination thereof:
   (a) a monomer, and optionally at least one comonomer;
   (b) an initiator, and optionally at least one co-initiator;
   (c) at least one crosslinking agent; and
   (d) at least one active agent deactivating agent.

In one embodiment, the TDD disposal system comprises at least one non-µ-opioid deactivating agent included at least in or on the adhesive coating or in or on the first and/or second substrate, or any combination thereof.

Another embodiment of the invention relates to a kit comprising a transdermal delivery device containing at least one active agent; and a TDD disposal system comprising at least a first rigid, non-planar substrate having an adhesive coating on at least one face.

A further embodiment of the invention relates to a kit comprising a transdermal delivery device comprising buprenorphine or any pharmaceutically acceptable form or derivative of buprenorphine; and a TDD disposal system comprising at least a first substrate having an adhesive coating on one face thereof.

An additional embodiment of the invention relates to a kit comprising a transdermal delivery device comprising fentanyl or any pharmaceutically acceptable form or derivative of fentanyl; and a TDD disposal system comprising at least a first substrate having an adhesive coating on one face thereof.

An additional embodiment of the invention relates to a kit comprising a transdermal delivery device comprising oxycodone or any pharmaceutically acceptable form or derivative of oxycodone; and a TDD disposal system comprising at least a first substrate having an adhesive coating on one face thereof.

Another embodiment of the invention relates to a TDD disposal system comprising at least a first substrate having an adhesive coating on one face thereof, where the adhesive is a silicone-based adhesive.

Another embodiment of the invention relates to a TDD disposal system comprising at least a first substrate having an adhesive coating on one face thereof, where the adhesive is an acrylate-based adhesive.

A further embodiment of the invention relates to a TDD disposal system and/or a kit comprising the same where the first substrate of the TDD disposal system exhibits one or more of the following properties:
(a) substantially solvent impermeable;
(b) substantially not solvent swellable;
(c) substantially tear resistant; and
(d) substantially cut resistant.

### BRIEF DESCRIPTION OF THE DRAWINGS

Figures 1A-1D each show a TDD disposal system in accordance with the present invention, having an area for immobilizing and isolating a single TDD, such as a patch, or multiple TDDs.

Figures 2A-2C show TDDs of the reservoir type, polymer matrix type, and drug-in-adhesive type, respectively.

Figure 3 shows a TDD disposal system containing a mixture of monomers, an initiator, and optionally a crosslinking agent and also a deactivating agent, optionally in a mixture with a degradation agent, within the same region.

Figure 4 shows a TDD disposal system having a deactivating agent, optionally in a mixture with a degradation agent, in a first region and a mixture of monomers, an initiator, and optionally a crosslinking agent in a second region.

Figures 5-10 each show the amount of buprenorphine (BUP) extractable from a TDD of Figure 2B and, thus, available for abuse, through extraction by distilled water, ethyl acetate, ethanol, ethyl ether, acetone, and methanol, respectively, and as the TDD was immobilized and isolated in a buprenorphine TDD disposal system of Figure 1B.

Figure 11 shows an occlusive TDD disposal system in accordance with the present invention bound in a book in the form of a leaf or multiple leaves, each with an area for immobilizing and isolating a single TDD, such as a patch, or multiple TDDs.

Figures 12A-D show an occlusive TDD disposal system in accordance with the present invention bound in a book in the form of multiple bi-fold leaves, each with an area for immobilizing and isolating a single TDD, such as a patch, or multiple TDDs.

Figure 13 shows a kit in accordance with the present invention including a box containing one or more transdermal delivery devices and a TDD disposal system in the form of a booklet attached to an outside face of the box.

Figure 14 shows a TDD disposal system in accordance with the present invention comprising two rigid non-planar substrates.

### DEFINITIONS

The phrase "transdermal delivery device" or "TDD" as used herein refers to any device that, when contacted with a patient's skin, can transdermally deliver a therapeutically effective amount of any biologically active agent, such as a pharmaceutical compound, *e.g*., an opioid, through the skin to the systemic circulation.

The term "opioid," when used in isolation herein, refers to a compound with µ-opioid receptor agonist activity.

As used herein, the terms "non-µ opioid" and "non-µ opioid agonist" refer to an active agent which binds, optionally stereospecifically, to any κ-opioid receptor, δ-opioid receptor, and/or ORL-1 opioid receptor, but significantly not to any µ-opioid receptor, and produces agonist activity.

As used herein, the terms "deactivating agent" is synonymous with the term "degradation agent" and includes "inactivating agent".

As used herein, the terms "non-µ opioid deactivating agent" refers to an agent which deactivates or degrades a non-µ opioid agonist but which does not deactivate or degrade an opioid agonist as defined herein.

It is, of course, to be understood that, unless specified to the contrary, any reference to any pharmaceutical compound throughout this disclosure includes not only that pharmaceutical compound, *i.e*., the so-called free form of that compound, but also pharmaceutically acceptable derivatives of that compound, *e.g*., pharmaceutically acceptable salts of that compound, base forms of that compound and their mixtures, and also mixtures of the free form and any or all derivative(s) and stereoisomers thereof.

The phrase "pharmaceutically acceptable salt," as used herein, *e.g*., for an opioid, refers to a salt formed from an acid and the basic nitrogen group of an opioid. Preferred salts include, but are not limited, to sulfate, citrate, acetate, oxalate, chloride, bromide, iodide, nitrate, bisulfate, phosphate, acid phosphate, isonicotinate, lactate, salicylate, acid citrate, tartrate, oleate, tannate, pantothenate, bitartrate, ascorbate, succinate, maleate, gentisinate, fumarate, gluconate, glucaronate, saccharate, formate, benzoate, glutamate, methanesulfonate, ethanesulfonate, benzenesulfonate, p-toluenesulfonate, and pamoate (*i.e*., 1,1'-methylene-bis-(2-hydroxy-3-naphthoate)) salts.

The term "base form" as used herein, *e.g*., for an opioid, refers to a salt prepared from an opioid having an acidic functional group, such as a carboxylic acid or sulfonic acid functional group, and a pharmaceutically acceptable inorganic or organic base. Suitable bases include, but are not limited to, hydroxides of alkali metals such as sodium, potassium, and lithium; hydroxides of alkaline earth metal such as calcium and magnesium; hydroxides of other metals, such as aluminum and zinc; ammonia, and organic amines, such as unsubstituted or hydroxy-substituted mono-, di-, or trialkylamines; dicyclohexylamine; tributyl amine; pyridine; N-methyl, N-ethylamine; diethylamine; triethylamine; mono-, bis-, or tris-(2-hydroxy-lower alkyl amines), such as mono-, bis-, or tris-(2-hydroxyethyl)amine, 2-hydroxy-tert-butylamine, or tris-(hydroxymethyl)methylamine, N, N,-di-lower alkyl-N-(hydroxy lower alkyl)-amines, such as N, N,-dimethyl-N-(2-hydroxyethyl)amine, or tri-(2-hydroxyethyl)amine; N-methyl-D-glucamine; and amino acids such as arginine, lysine, and the like.

The term "substantially," as used herein, when modifying an adjective or adjective phrase immediately succeeding it, should be understood to mean that adjective or adjective phrase applies to at least about a 95% level, preferably to at least about a 98% level, more preferably to at least a 99% level, for example to at least about a 99.9% level, with respect to the noun or pronoun that the adjective or adjective phrase modifies. Alternately, the term "substantially" may be used interchangeably with the term "completely."

The phrases "substantially not" or "substantially no," as used herein, when modifying an adjective or adjective phrase immediately succeeding it, should be understood to mean that adjective or adjective phrase applies to not more than about a 5% level, preferably to not more than about a 2% level, more preferably to not more than about a 1% level, for example to not more than about a 0.1 % level, with respect to the noun or pronoun that the adjective or adjective phrase modifies. Alternately, the phrases "substantially not" and "substantially no" may be used interchangeably with the phrases "completely not" and "completely no," respectively.

The term "about", as used herein, when modifying a numerical value or range, should be understood to mean plus or minus 10% of the numerical value or range.

As used herein, a "copolymer" includes a polymer comprising at least two different monomeric subunits. Thus, a polymeric chain made up of three different monomers (also known as a terpolymer) is included within the term "copolymer," as are polymer chains containing more than three different monomeric units.

As used herein, the term "oligomer" includes compounds comprising repeating monomeric units, like a polymer or copolymer, but with a low molecular weight such that the compound would not be considered to be polymeric.

### DETAILED DESCRIPTION OF THE PREFERRED EMBODIMENTS

This applications claims priority to U.S. Provisional Patent Application No. 60/387,800, filed June 10, 2002, which is hereby incorporated by reference in its entirety by express reference hereto.

The disposal system of the invention may be used to dispose of any type of TDD, regardless of the construction or form of that device and regardless of the active agent or agents contained within the device. Active agents which may typically be administered by a TDD include, but are not limited to, opioids, non-µ-opioids, anti-emetics (scopolamine), cardiovascular agents (nitrates and clonidine), hormones (estrogen and testosterone), nicotine, vitamins, dietary supplements. For purposes of illustration herein, reference will be made primarily to TDDs containing an opioid analgesic as the pharmaceutically active agent.

### 1. Transdermal Delivery Devices

Typical TDDs include a pharmaceutical compound, such as at least one opioid, and, optionally, at least one opioid antagonist in an amount sufficient to inhibit the euphoric effect of the opioid in the event that the opioid and the opioid antagonist are administered, *e.g*., orally, intravenously, buccally, nasally, parenterally, rectally, and/or vaginally, to a mammal, usually to a human. When contacted with a patient's skin, such typical TDDs allow for the transdermal administration of the opioid but either (a) permit the transdermal administration of only a small amount of the opioid antagonist that is ineffective for inhibiting the analgesic effect of the opioid or (b) do not permit the transdermal administration of the opioid antagonist. However, if such TDDs are used to deliver the opioid via a route other than transdermal, *e.g*., such as intravenous, buccal, nasal, oral, parenteral, rectal, and/or vaginal, then the opioid antagonist blunts or inhibits the euphoric effect of the opioid. Preferably, such TDDs inhibit the euphoric effect of the opioid if used other than transdermally regardless of whether the device has previously been used appropriately for treating or preventing pain.

While not required, a TDD may be constructed so as to deter an abuser from either (a) separating the opioid antagonist from the opioid or (b) isolating the opioid from the TDD, and then self-administering the opioid via another route, such as, but not limited to, orally, parenterally, nasally, intravenously, buccally, or by inhalation of vapors, *i.e.,* a route of administration that can result in a quick euphoric rush, also known as the "burst," that abusers prefer. For example, if an abuser tries to extract the opioid from the TDD by placing it in a solvent, then the opioid antagonist would also be extracted, providing a mixture of the opioid and the opioid antagonist. If a mixture of the opioid and opioid antagonist is administered via a route other than the intended transdermal route, then the opioid antagonist would exert its antagonistic effect to inhibit the euphoric effect of the opioid.

Any device known to those skilled in the art for transdermally delivering a therapeutic agent to a patient can qualify as a TDD. For example, the TDD can be a reservoir-type TDD, a polymer-matrix type TDD, or a drug-in-adhesive type TDD (*See, e.g.,* H.S. Tan and W R. Pfister, Pressure Sensitive Adhesives for Transdermal Drug Delivery Systems, PSTT, vol. 2, no. 2, Feb. 1999, pp. 60-69, the disclosure of which is incorporated herein by reference). The TDD is designed so that when contacted with the patient's skin, an analgesically effective amount of the opioid is transdermally administered to the patient. But the opioid antagonist either remains in the TDD and is not administered to the patient or is administered to the patient in an amount insufficient to inhibit the analgesic effect of the opioid.

A reservoir-type TDD typically comprises a reservoir, usually a liquid, located between an impermeable backing film and a rate-controlling membrane that is covered with a pressure-sensitive adhesive skin-contacting layer. The reservoir, which can be a solution or a dispersion, contains the opioid and the opioid antagonist. The TDD is supported by the impermeable backing film and the adhesive surface is protected by a release liner. To administer the opioid, the release liner is removed to expose the pressure-sensitive adhesive and the pressure-sensitive adhesive is contacted with the skin. The opioid is permeable through the rate-controlling membrane, and penetrates through it and the adhesive, contacts the skin, and then penetrates the skin. The delivery rate of the opioid is usually determined by the rate that the opioid penetrates the rate-controlling membrane. Preferably, the pressure-sensitive adhesive does not adversely affect the delivery rate of and does not chemically react with the opioid. The delivery rate is such that an analgesically effective amount of the opioid is delivered to the patient. In contrast to the opioid, however, the opioid antagonist, which may be present anywhere in the reservoir, preferably does not penetrate the rate-controlling membrane or, if it does, does so in an amount insufficient to inhibit the analgesic effect of the opioid.

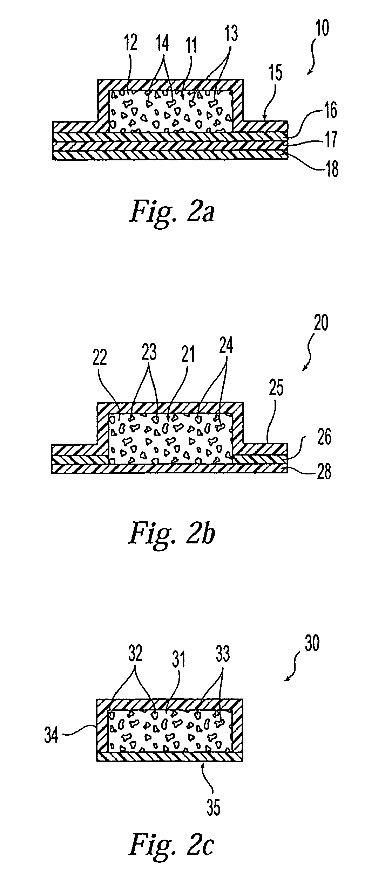

Figure 2A depicts one embodiment of a reservoir-type TDD. The TDD 10 comprises a reservoir 11, typically in the form of a solution or a dispersion 12, having dispersed therein an opioid 13 and an opioid antagonist 14. The reservoir 11 is disposed between an impermeable backing film 15, a rate-controlling membrane 16, and a pressure-sensitive adhesive 17. A release liner 18 is applied to the pressure-sensitive adhesive layer 17, and is removed prior to use. Preferably, the opioid and the opioid antagonist are dispersed throughout the reservoir, although uniform dispersion is not necessary.

A variation of the reservoir-type TDD is the polymer-matrix design. In the polymer-matrix design, the opioid and the opioid antagonist are dispersed in a polymer matrix that controls the delivery rate of the opioid. Similar to the liquid-reservoir design, the polymer-matrix reservoir is supported on a impermeable backing layer. Rather than having a continuous adhesive layer, however, the polymer-matrix design usually includes a peripheral ring of adhesive located around the edge of the patch. A release liner protects the adhesive surface and the surface of the polymer matrix. To administer the opioid, the release liner is removed to expose the polymer matrix and the ring of pressure-sensitive adhesive, and the device is contacted with the skin. The ring of adhesive holds the device against the skin so that the polymer matrix directly contacts the skin. When the polymer matrix is contacted with the skin, the opioid diffuses out of the polymer matrix, contacts the patient's skin, and penetrates the skin. The delivery rate of the opioid agonist is usually determined by the rate of diffusion of the opioid out of the polymer matrix. The delivery rate is such that an analgesically effective amount of the opioid is delivered to the patient. The opioid antagonist, which may be present anywhere in the polymer matrix, on the other hand, either does not diffuse out of the polymer matrix or, if it does, does so in an amount insufficient to inhibit the analgesic effect of the opioid.

Figure 2B depicts a typical polymer-matrix TDD embodiment of the invention. The TDD 20 comprises a reservoir 21 in the form of a polymer matrix 22, having dispersed therein an opioid 23 and an opioid antagonist 24. Preferably, the opioid and the opioid antagonist are dispersed throughout the polymer matrix, although uniform dispersion is not necessary. The polymer matrix 21 is supported on an impermeable backing layer 25 and has a peripheral ring of adhesive 26 located around the edge of the patch. A release liner 28 is applied to the peripheral ring of adhesive 26 and polymer matrix 22 and is removed prior to use.

The drug-in-adhesive type TDD comprises the opioid agonist and the opioid antagonist dispersed directly in a pressure-sensitive adhesive matrix. The adhesive matrix is typically supported on the topside with an impermeable backing film and on the side that faces the skin with an impermeable release liner. To administer the opioid, the release liner is removed to expose the adhesive matrix, and the device is contacted with the skin. The adhesive matrix functions to adhere the device to the skin and, typically, to control the delivery rate of the opioid. Similar to the polymer-matrix design, the drug-in-adhesive design allows the opioid to diffuse out of the adhesive matrix, contact the patient's skin, and penetrate the skin. The delivery rate of the opioid is usually determined by the rate of diffusion of the opioid out of the adhesive matrix. The delivery rate is such that an analgesically effective amount of the opioid is delivered to the patient. The opioid antagonist, on the other hand, which may be present anywhere in the adhesive matrix, does not diffuse out of the adhesive matrix or does so in an amount insufficient to inhibit the analgesic effect of the opioid.

Figure 2C depicts a typical drug-in-adhesive TDD embodiment of the invention. The TDD 30 comprises an adhesive matrix 31 having dispersed there through an opioid 32 and an opioid antagonist 33. Preferably, the opioid and the opioid antagonist are dispersed throughout the adhesive matrix, although uniform dispersion is not necessary. The adhesive matrix 31 is supported on an impermeable backing layer 34 and has an impermeable release liner 35 on the side that faces the skin which is removed prior to use.

The reservoir type, polymer matrix type, and the drug-in-adhesive type TDDs are well-known to those skilled in the art (*See, e.g.,* H. Tan and W. Pfister, "Pressure Sensitive Adhesives for Transdermal Drug Delivery Systems," PSTT, vol. 2, February 1999, the disclosure of which is incorporated herein by reference). In one preferred embodiment, the TDD disposal system according to the invention advantageously immobilizes and isolates a polymer matrix type TDD and/or a drug-in-adhesive type TDD to prevent, control, or inhibit misuse and/or abuse of the active agent contained in the TDD.

Any rate-controlling membrane known to those skilled in the art can be used in the TDD of the invention. It is preferable to select a membrane which does not allow any, or any detectable amount, of the opioid antagonist to penetrate therethrough, particularly in those instances in which the opioid antagonist can penetrate a patient's skin. Suitable materials for the rate-controlling membranes include, but are not limited to, polyethylene; polypropylene; ethylene/propylene copolymers; ethylene/ethylacrylate copolymers; ethylene/vinyl acetate copolymers; polyacrylates; polymethacrylates; silicone elastomers; medical-grade polydimethylsiloxanes; neoprene rubber; polyisobutylene; chlorinated polyethylene; polyvinyl chloride; vinyl chloride-vinyl acetate copolymer; polymethacrylate polymer (hydrogel); polyvinylidene chloride; poly(ethylene terephthalate); butyl rubber; epichlorohydrin rubbers; ethylene-vinyl alcohol copolymer; ethylene-vinyloxyethanol copolymer; silicone copolymers, for example polysiloxane-polycarbonate copolymers, polysiloxane-polyethyleneoxidecopolymers, polysiloxane-polymethacrylate copolymers, polysiloxane-alkylene copolymers (*e.g*., polysiloxane-ethylene copolymers), polysiloxane-alkylenesilane copolymers (*e.g*., poly(siloxane-co-ethylenesilane), and the like; cellulose polymers, for example, methyl or ethyl cellulose, hydroxypropyl methyl cellulose, and cellulose esters; polycarbonates; polytetrafluoroethylene; starches; gelatin; natural and synthetic gums; any other natural or synthetic polymer or fiber; and combinations thereof.

The backing layer can be any suitable material that is impermeable to the contents of the reservoir compartment, the polymer matrix, or the adhesive matrix. Suitable materials for backing films are well known to those skilled in the art and include, but are not limited to, occlusive polymers such as polyurethane, polyesters such as poly(ethylene phthalate), polyether amide, copolyester, polyisobutylene, polyesters, high and low density polyethylene, polypropylene, polyvinylchloride, metal foils, and metal foil laminates of suitable polymer films.

Suitable materials for the polymer matrix are well known to those skilled in the art and include, but are not limited to, polyethylene; polypropylene; ethylene/propylene copolymers; ethylene/ethylacrylate copolymers; ethylene/vinyl acetate copolymers; silicone elastomers, especially the medical-grade polydimethylsiloxanes; neoprene rubber; polyisobutylene; chlorinated polyethylene; polyvinyl chloride; vinyl chloride-vinyl acetate copolymer; polymethacrylate polymer (hydrogel); polyvinylidene chloride; poly(ethylene terephthalate); butyl rubber; epichlorohydrin rubbers; ethylene-vinyl alcohol copolymer; ethylene-vinyloxyethanol copolymer; silicone copolymers, for example, polysiloxane-polycarbonate copolymers, polysiloxane-polyethyleneoxide copolymers, polysiloxane-polymethacrylate copolymers, polysiloxane-alkylene copolymers (*e.g*., polysiloxane-ethylene copolymers), polysiloxane-alkylenesilane copolymers (*e.g*., poly(siloxane-co-ethylenesilane), and the like; cellulose polymers, for example methyl or ethyl cellulose, hydroxypropyl methyl cellulose, and cellulose esters; polycarbonates; polytetrafluoroethylene; and combinations thereof. Preferably, the polymer matrix has a glass transition temperature below room temperature. The polymer can, but need not necessarily, have a non-zero degree of crystallinity at room temperature. Cross-linking monomeric units or sites can be incorporated into the polymers. For example, cross-linking monomers can be incorporated into polyacrylate polymers. The cross-linking monomers provide sites for cross-linking the polymer matrix after microdispersing the opioid and opioid antagonist into the polymer. Known cross-linking monomers for polyacrylate polymers include, but are not limited to, polymethacrylic esters of polyols such as butylene diacrylate and dimethacrylate, trimethylol propane trimethacrylate, and the like. Other monomers that provide cross-linking sites include allyl acrylate, allyl methacrylate, diallyl maleate, and the like. It is preferable to select a polymer matrix which does not allow any, or any detectable amount, of the opioid antagonist to diffuse out of it, particularly in those instances in which the opioid antagonist can penetrate a patient's skin.

Suitable materials for the pressure-sensitive adhesive matrix are well known to those skilled in the art and include, but are not limited to, polyisobutylenes, polysiloxanes, and polyacrylate copolymers (polyacrylic esters), natural rubber/karaya gum-based adhesives, hydrogels, hydrophilic polymers, and polyurethanes such as those described in H. Tan and W. Pfister, "Pressure Sensitive Adhesives for Transdermal Drug Delivery Systems," PSTT, vol. 2, February 1999, the disclosure of which is incorporated herein by reference. The adhesive may further include modifying monomers, tackifiers, plasticizers, fillers, waxes, oils, and other additives to impart the desired adhesive properties. *Id.* It is preferable to select a pressure-sensitive adhesive matrix that does not allow any, or any detectable amount, of the opioid antagonist to diffuse out it, particularly in those instances in which the opioid antagonist can penetrate a patient's skin.

Preferably, the size of the device can vary from about 1 cm² to greater than 200 cm² and typically is between about 5-50 cm². Methods for manufacturing TDDs are well known to those skilled in the art.

Examples of devices useful in transdermal delivery devices include, but are not limited to, those described in U.S. Patent Nos. 4,806,341; 5,069,909; 5,236,714; 5,240,711; 5,718,914; 5,902,603; 5,968,547; 6,162,456; and 6,344,212, the disclosures of which are incorporated herein by reference.

The TDD can optionally include one or more penetration enhancers, which increase the rate at which the opioid penetrates through the patient's skin. Preferably, the penetration enhancer does not enhance the penetration of the opioid antagonist through the skin. The penetration enhancer should penetrate the rate-controlling membrane or diffuse out of the polymer matrix or adhesive matrix so that it can contact the patient's skin and improve penetration of the opioid through the patient's skin. Suitable penetration enhancers for use in the TDDs and methods of the invention include, but are not limited to, C₂-C₄ alcohols such as ethanol and isopropanol, polyethylene glycol monolaurate, polyethylene glycol-3-laurarnide, dimethyl lauramide, sorbitan trioleate, fatty acids, esters of fatty acids having from about 10 to about 20 carbon atoms, monoglycerides or mixtures of monoglycerides of fatty acids having a total monoesters content of at least 51 % where the monoesters are those with from 10 to 20 carbon atoms, and mixtures of mono-, di- and tri-glycerides of fatty acids. Suitable fatty acids include, but are not limited to lauric acid, myristic acid, stearic acid, oleic acid, linoleic acid and palmitic acid. Monoglyceride permeation enhancers include glycerol monooleate, glycerol monolaurate, and glycerol monolinoleate, for example. Examples of penetration enhancers useful in the methods of the invention include, but are not limited to those described in U.S. Patent Nos. 3,472,931; 3,527,864; 3,896,238; 3,903,256; 3,952,099; 3,989,816; 4,046,886; 4,130,643; 4,130,667; 4,299,826; 4,335,115; 4,343,798; 4,379,454; 4,405,616; 4,746,515; 4,316,893; 4,405,616; 4,060,084, 4,379,454; 4,560,5534,863,952; 4,863,970; 4,879,275; 4,940,586; 4,960,771; 4,973,968; 5,066,648; 5,164,406; 5,227,169; 5,229,130; 5,238,933; 5,308,625; 4,326,566; 5,378,730; 5,420,106; 5,641,5045,716,638; 5,750,137;5,785,991; 5,837,289; 5,834,468; 5,882,676; 5,912,009; 5,952,000; 6,004,578; and Idson, Percutaneous Absorption, J. Pharm. Sci. vol. 64, no. b6, June 1975, pp. 901-924, the disclosures of which are incorporated herein by reference.

The TDD can further comprise other additives conventionally used in therapeutic products. For example, the TDD can also include one or more preservatives or bacteriostatic agents, *e.g*., methyl hydroxybenzoate, propyl hydroxybenzoate, chlorocresol, benzalkonium chlorides, and the like; or other active ingredients such as antimicrobial agents, particularly antibiotics; anesthetics; other analgesics; and antipruritic agents.

Any opioid or non-µ-opioid, a pharmaceutically acceptable salt thereof, a base form thereof, or mixture of any combination of such opioids and/or their derivatives, that is known in the art can be contained in the TDD. Opioids believed to have at least some µ-opioid receptor agonist activity (and optionally at least some agonist activity also at one or more of the κ-opioid receptor, the δ-opioid receptor, and the ORL-1 receptor) include, but are not limited to, alfentanil, allylprodine, alphaprodine, anileridine, benzylmorphine, bezitramide, buprenorphine, butorphanol, clonitazene, codeine, desomorphine, dextromoramide, dezocine, diampromide, diamorphone, dihydrocodeine, dihydromorphine, dihydromorphone, dihydroisomorphine, dimenoxadol, dimepheptanol, dimethylthiambutene, dioxaphetyl butyrate, dipipanone, eptazocine, ethoheptazine, ethylmethylthiambutene, ethylmorphine, etonitazene, etorphine, dihydroetorphine, fentanyl, heroin, hydrocodone, hydromorphone, hydromorphodone, hydroxypethidine, isomethadone, ketobemidone, levorphanol, levophenacylmorphan, lofentanil, meperidine, meptazinol, metazocine, methadone, metopon, morphine, myrophine, narceine, nicomorphine, norlevorphanol, normethadone, nalorphine, nalbuphene, normorphine, norpipanone, opium, oxycodone, oxymorphone, pantopon, papaveretum, paregoric, pentazocine, phenadoxone, phendimetrazine, phendimetrazone, phenomorphan, phenazocine, phenoperidine, piminodine, piritramide, propheptazine, promedol, properidine, propoxyphene, propylhexedrine, sufentanil, tilidine, tramadol, and mixtures thereof. Non-µ-opioids include, but are not limited to, ORL-1-specific opioid agonists, such as nociceptin, deltorphin, and the like, and mixtures thereof. In a preferred embodiment, the opioid includes buprenorphine, pharmaceutically acceptable salts thereof, base forms thereof, fentanyl, pharmaceutically acceptable salts thereof, base forms thereof, oxycodone, pharmaceutically acceptable salts thereof, base forms thereof, and any combination of such opioids and/or their derivatives.

In certain preferred embodiments, the opioid agonist includes hydrocodone, morphine, hydromorphone, oxycodone, codeine, levorphanol, meperidine, methadone, oxymorphone, buprenorphine, fentanyl, dipipanone, heroin, tramadol, etorphine, dihydroetorphine, butorphanol, levorphanol, pharmaceutically acceptable salts thereof, base forms thereof, and any and all mixtures thereof. More preferably the opioid agonist includes oxycodone, hydrocodone, fentanyl, buprenorphine, pharmaceutically acceptable salts thereof, base forms thereof, and any and all mixtures thereof. Most preferably, the opioid agonist includes buprenorphine, pharmaceutically acceptable salts thereof, base forms thereof, fentanyl, pharmaceutically acceptable salts thereof, base forms thereof, and any combination of such opioids and/or their derivatives.

Preferably, especially for passive patches, the opioid is a free opioid, *i.e*., not a pharmaceutically acceptable salt form of the opioid. However, for patches that use iontophoresis to facilitate penetration of the skin by the opioid, a pharmaceutically acceptable salt form of the opioid is preferred.

Alternately, instead of, or in addition to, an opioid agonist, the TDD may contain a pharmacologically active agent that is capable of inducing a desired biological or pharmacological effect, which may include, but is not limited to, (1) affecting living processes; (2) having a prophylactic effect on the patient and preventing an undesired effect, such as preventing an infection; (3) alleviating a condition caused by, or being a symptom of, a disease, *e.g*., alleviating pain or inflammation; and/or (4) either alleviating, reducing, or completely eliminating the disease, condition, or symptom from the patient. The effect of the active agent may be local, such as for providing an anaesthetic effect, or it may be systemic or a combination thereof. General categories of active agents can, in one embodiment, include, but are not limited to: ACE inhibitors; adenohypophyseal hormones; adrenergic neuron blocking agents; adrenocortical steroids; inhibitors of the biosynthesis of adrenocortical steroids; alpha-adrenergic agonists; alpha-adrenergic antagonists; selective alpha-two-adrenergic agonists; androgens; anti-addictive agents; antiandrogens; antiinfectives, such as antibiotics, antimicrobials, and antiviral agents; analgesics and analgesic combinations; anorexics; antihelmintics; antiarthritics; antiasthmatic agents; anticonvulsants; antidepressants; antidiabetic agents; antidiarrheals; antiemetic and prokinetic agents; antiepileptic agents; antiestrogens; antifungal agents; antihistamines; antiinflammatory agents; antimigraine preparations; antimuscarinic agents; antinauseants; antineoplastics; antiparasitic agents; antiparkinsonism drugs; antiplatelet agents; antiprogestins; antipruritics; antipsychotics; antipyretics; antispasmodics; anticholinergics; antithyroid agents; antitussives; azaspirodecanediones; sympathomimetics; xanthine derivatives; cardiovascular preparations, including potassium and calcium channel blockers, alpha blockers, beta blockers, and antiarrhythmics; antihypertensives; diuretics and antidiuretics; vasodilators, including general coronary, peripheral, and cerebral; central nervous system stimulants; vasoconstrictors; cough and cold preparations, including decongestants; hormones, such as estradiol and other steroids, including corticosteroids; hypnotics; immunosuppressives; muscle relaxants; parasympatholytics; psychostimulants; sedatives; tranquilizers; nicotine and acid addition salts thereof; benzodiazepines; barbituates; benzothiadiazides; beta-adrenergic agonists; beta-adrenergic antagonists; selective beta-one-adrenergic antagonists; selective beta-two-adrenergic antagonists; bile salts; agents affecting volume and composition of body fluids; butyrophenones; agents affecting calcification; catecholamines; cholinergic agonists; cholinesterase reactivators; dermatological agents; diphenylbutylpiperidines; ergot alkaloids; ganglionic blocking agents; hydantoins; agents for control of gastric acidity and treatment of peptic ulcers; hematopoietic agents; histamines; 5-hydroxytryptamine antagonists; drugs for the treatment of hyperlipiproteinemia; laxatives; methylxanthines; moncamine oxidase inhibitors; neuromuscular blocking agents; organic nitrates; pancreatic enzymes; phenothiazines; prostaglandins; retinoids; agents for spasticity and acute muscle spasms; succinimides; thioxanthines; thrombolytic agents; thyroid agents; inhibitors of tubular transport of organic compounds; drugs affecting uterine motility; vitamins; and the like; or a combination thereof.

Alternately, instead of, or in addition to, an opioid agonist, the TDD can contain an active component that may include, but is not limited to, flurogestone acetate, hydroxyprogesterone, hydroxyprogesterone acetate, hydroxyprogesterone caproate, medroxy-progesterone acetate, norethindrone, norethindrone acetate, norethisterone, norethynodrel, desogestrel, 3-keto desogestrel, gestadene, levonorgestrel, estradiol, estradiol benzoate, estradiol valerate, estradiol cyprionate, estradiol decanoate, estradiol acetate, ethynyl estradiol, estriol, estrone, mestranol, betamethasone, betamethasone acetate, cortisone, hydrocortisone, hydrocortisone acetate, corticosterone, fluocinolone acetonide, prednisolone, prednisone, triamcinolone, aldosterone, androsterone, testosterone, methyl testosterone, or a combination thereof.

Alternately, instead of, or in addition to, an opioid agonist, the TDD can contain an active component that may include, but is not limited to: a) corticosteroids, *e.g*., cortisone, hydrocortisone, prednisolone, beclomethasone propionate, dexamethasone, betamethasone, flumethasone, triamcinolone, triamcinolone acetonide, fluocinolone, fluocinolone acetonide, fluocinolone acetate, clobetasol propionate, or the like, or a combination thereof; b) analgesic anti-inflammatory agents, *e.g*., acetaminophen, mefenamic acid, flufenamic acid, indomethacin, diclofenac, diclofenac sodium, alclofenac, ibufenac, oxyphenbutazone, phenylbutazone, ibuprofen, flurbiprofen, ketoprofen, salicylic acid, methylsalicylate, acetylsalicylic acid, 1-menthol, camphor, slindac, tolmetin sodium, naproxen, fenbufen, or the like, or a combination thereof; c) hypnotic sedatives, *e.g*., phenobarbital, amobarbital, cyclobarbital, lorazepam, haloperidol, or the like, or a combination thereof; d) tranquilizers, *e.g*., fulphenazine, thioridazine, diazepam, flurazepam, chlorpromazine, or the like, or a combination thereof; e) antihypertensives, *e.g*., clonidine, clonidine hydrochloride, bopinidol, timolol, pindolol, propranolol, propranolol hydrochloride, bupranolol, indenolol, bucumolol, nifedipine, bunitrolol, or the like, or a combination thereof; f) hypotensive diuretics, *e.g*., bendroflumethiazide, polythiazide, methylchlorthiazide, trichlormethiazide, cyclopenthiazide, benzyl hydrochlorothiazide, hydrochlorothiazide, bumetanide, or the like, or a combination thereof; g) antibiotics, *e.g*., penicillin, tetracycline, oxytetracycline, metacycline, doxycycline, minocycline, fradiomycin sulfate, erythromycin, chloramphenicol, or the like, or a combination thereof; h) anesthetics, *e.g*., lidocaine, benzocaine, ethylaminobenzoate, or the like, or a combination thereof; i) antimicrobial agents, e.g., benzalkonium chloride, nitrofurazone, nystatin, sulfacetamide, clotriamazole, or the like, or a combination thereof; j) antifungal agents, *e.g*., pentamycin, amphotericin B, pyrrol nitrin, clotrimazole, or the like, or a combination thereof; k) vitamins, *e.g*., vitamin A, ergocalciferol, cholecalciferol, octotriamine, riboflavin butyric acid ester, or the like, or a combination thereof; 1) antiepileptics, *e.g*., nitrazepam, meprobamate, clonazepam, or the like, or a combination thereof; m) antihistamines, *e.g*., diphenhydramine hydrochloride, chlorpheniramine, diphenylimidazole, or the like, or a combination thereof; n) antitussives, *e.g*., dextromethorphan, terbutaline, ephedrine, ephedrine hydrochloride, or the like, or a combination thereof; o) sex hormones, *e.g*., progesterone, estradiol, estriol, estrone, or the like, or a combination thereof; p) antidepressants, *e.g*., doxepin; q) vasodilators, *e.g*., nitroglycerin, isosorbide nitrate, nitroglycol, pentaerythritol tetranitrate, dipyridamole, or the like, or a combination thereof; r) other drugs, *e.g*., 5-fluorouracil, dihydroergotamine, desmopressin, digoxin, methoclopramide, domperidone, scopolamine, scopolamine hydrochloride, or the like, or a combination thereof; or the like; or a combination thereof.

The effective amount of active agent present in the TDD will depend on the specific active agent, the type of device, the materials used to manufacture the device, and the duration for which the active agent will be delivered to the patient. When the active agent is an opioid, the analgesically effective amount of opioid present in the TDD, however, is typically from about 0.1 to about 500 mg, preferably from about 1 to about 100 mg, and more preferably from about 1 to about 50 mg. It is well within the purview of one skilled in the art to readily determine the analgesically effective amount of opioid or non-p-opioid needed for a particular indication.

### 2. TDD disposal systems

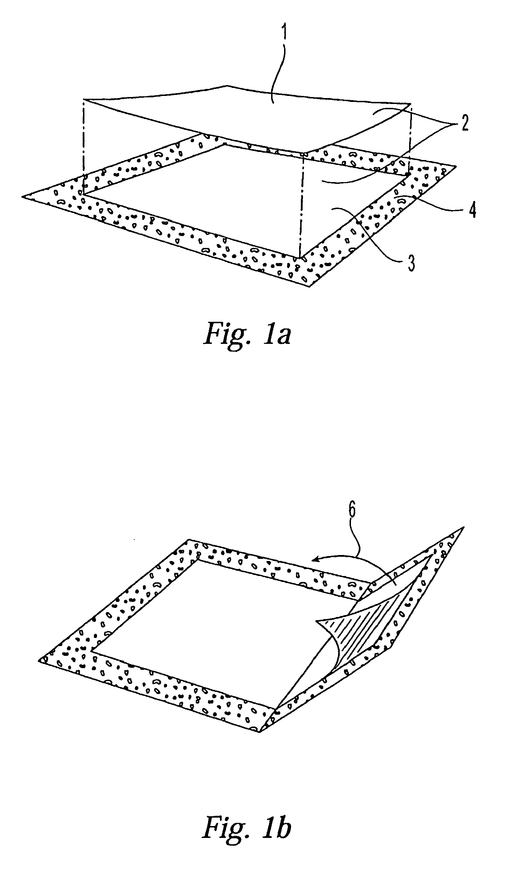

TDD disposal systems according to the invention preferably contain at least one substrate on which an adhesive is coated. There may be at least one substrate which may be flexible *(i.e.,* readily capable of being folded upon itself without substantially losing its mechanical integrity) or rigid (*i.e*., which can be either mechanically or physically resistant to being folded upon itself, or not foldable upon itself without substantially losing its mechanical integrity, either manually or mechanically). Flexible substrates can be represented, *e.g*., as shown in Figure 1B (for single TDD disposal systems) and 1D (for multiple TDD disposal systems). Rigid substrates can be represented, *e.g*., as shown in Figure 1A (for single TDD disposal systems) and 1C (for multiple TDD disposal systems). The flexibility or rigidity of at least one substrate according to the invention can be influenced not only by the chemical nature of the substrate material, but also, *inter alia,* by the dimensions (thickness, length, and/or width) of the substrate material.

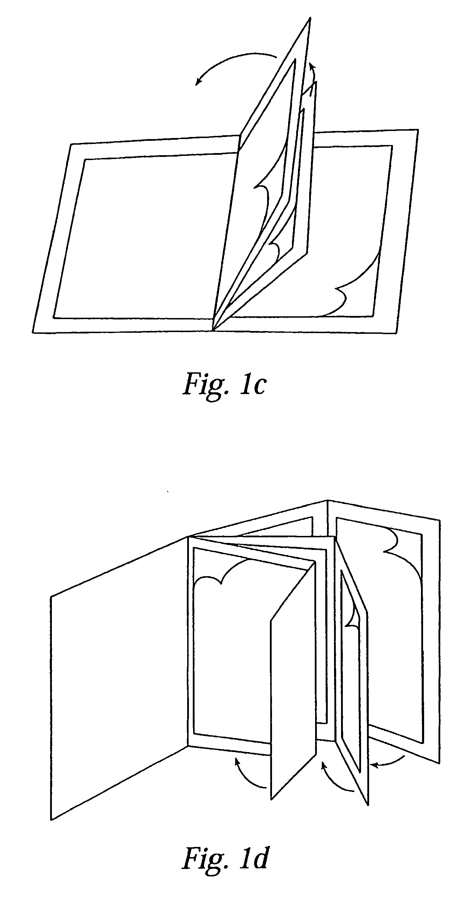

As shown in Figure 1A, the TDD disposal system according to the invention includes an outer layer (1) and an inner layer (4), which are joined by an adhesive (3) covering a first portion of the inner layer. The combination of the inner and outer layers forms a closed immobilization and/or sealing region (2) between the outer layer facing-side of the inner layer and the inner layer-facing side of the outer layer. As shown further in Figure 1B, the inner and outer layers are preferably joined in such a way as to form a sealable opening between the two layers. As shown in Figure 1B, the opening is preferably sealed by a flap (6) containing the adhesive (3) covering at least a portion of the flap that is used to seal the opening. Figures 1C and 1D show multi-substrate booklet analogs of the single-substrate TDD disposal systems shown in Figures 1A and 1B, respectively.

If there is only one substrate on which an adhesive is coated, it can preferably be flexible, so as to be capable of folding over onto itself in order to immobilize and isolate a TDD placed therein. Alternately, if there is only one substrate on which an adhesive is coated, a second complementary substrate may be present, which second substrate should preferably be capable of being irreversibly adhered to at least one adhesive-coated substrate. In this alternate embodiment, the adhesive-coated substrate and the second complementary substrate may be flexible or rigid, as desired.

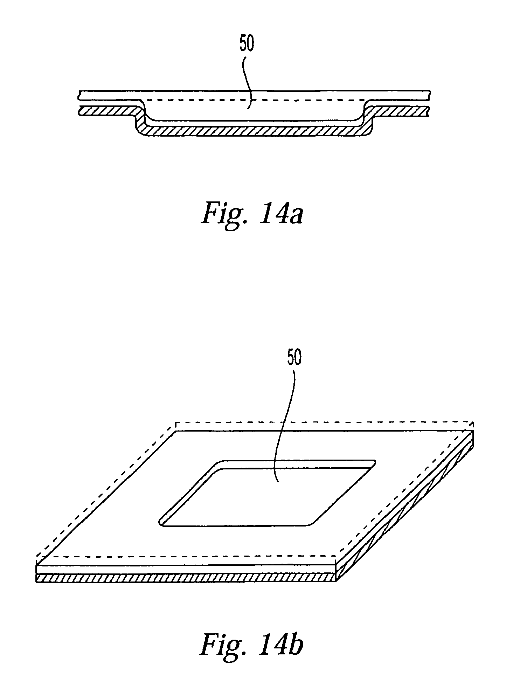

In certain embodiments, the substrate or substrates may be sufficiently rigid to render the substrate incapable of being folded upon itself without damaging or cracking the substrate. In accordance with these embodiments, the disposal system may comprise one or more rigid substrates which may be either planar or non-planar. As illustrated in Figures 14A and 14B, the non-planar substrates may preferably be configured and dimensioned to provide a recess 50 in one face for accepting the TDD. Depending on the thickness of the substrate and the depth of the recess, the face of the substrate which is opposite to the recess may be either planar or non-planar. As shown in the embodiment illustrated in Figure 14A, the substrate may have a recess on one face and a corresponding raised surface on the opposite face. Preferably, adhesive coating and any optional components present are disposed at least in or on the substrate inside the recess as well as over another portion of the face of the substrate. Additionally, two substrates each having a recess may be provided. In use, the TDD may be placed substantially within the recess of a first substrate and a second substrate may be aligned and placed in contact with the first substrate such that the recess of the second substrate is substantially aligned over the TDD and the TDD, the first substrate and the second substrate are irreversibly adhered together. The recess 50 may have a depth which is less than, equal to or more than the thickness of the TDD. Preferably, if there is one substrate, the thickness of the recess is about equal to or greater than the thickness of the TDD. If there are two substrates, either one or both may be provided with recesses. In such embodiments, the total depth of the recess or recesses may be less than, equal to or more than the thickness of the TDD, preferably about equal to the thickness of the TDD.

In certain embodiments, the substrates may additionally comprise one or more protruding members which are sufficiently rigid and/or sharp so as to penetrate into a TDD which is placed therein. Such embodiments are particularly advantageous for piercing the reservoir of reservoir type TDD's in order to allow the active agent to escape the reservoir more readily and interact with the substrate, the adhesives and any other optional components which are present.

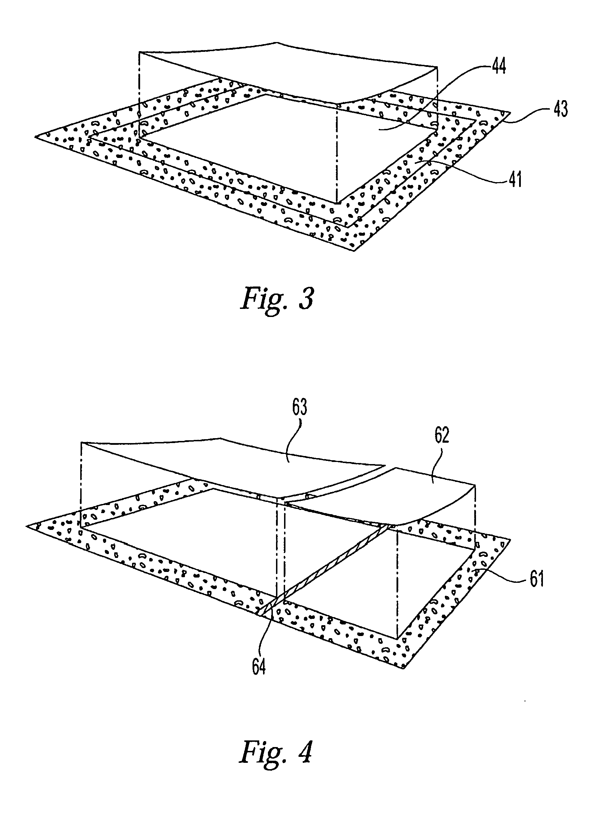

Figure 3 illustrates an alternate embodiment of the present invention. The article is a TDD disposal system having one or more of initiators (and optionally coinitiators), monomers (and optionally comonomers), crosslinking agents, and deactivating agents (for the TDD active component(s)) disposed within the immobilization and/or sealing region of the TDD disposal system. Specifically, the TDD disposal system according to the invention includes an adhesive layer (optionally containing one or more of the additional components) (41) having a transfer side and an opposite side. The article further includes an outer layer (43) having an adhesive layer-facing side and an opposite side, wherein the adhesive layer is joined to the outer layer to form an immobilization and/or sealing region (44) between the opposite side of the adhesive layer and the adhesive layer-facing side of the outer layer. Preferably, each of the layers of the TDD disposal system of the invention are tear-resistant.

Figure 4 illustrates an additional embodiment of the TDD disposal system of the invention, having a transfer side and an opposite side, and optionally an adhesive (61) disposed on the perimeter of the transfer side. The transfer side comprises a first (62) and second'(63) region, which can optionally be separated from one another by a membrane or impermeable barrier (64). Either the first region or the second region, or both, has at least one of the aforementioned components disposed therein. Preferably, if both the first and second regions contain at least one of the aforementioned components, then the component(s) present is(are) not exactly the same in each region. The TDD disposal system optionally also includes a release layer covering the adhesive, and the release layer is optionally peelable.

The substrate(s) that is(are) included in the TDD disposal system according to the invention may preferably include, but is(are) not limited to, tough, tear-resistant (co)polymers and/or (co)polymer composites. The substrate (co)polymers can be amorphous or may advantageously be oriented in a manner including, but not limited to, uniaxially, biaxially, multiaxially, planar-wise, chain-folded crystalline, chain-extended crystalline, chain-extended non-crystalline, liquid crystalline, fibrillar, lamellar, spherulitic, or the like, or any combination thereof. Examples of substrates for use in the TDD disposal system according to the invention may include, but are not limited to: polyesters, *e.g.,* poly(ethylene terephthalate), poly(butylene terephthalate), poly(ester-ether) copolymers, poly(ester-amide) copolymers, or the like; polycarbonates; polyurethanes, *e.g*., poly(esterurethane)s, poly(ether-urethane)s, poly(ether-urethane-urea)s, poly(ester-urethane-urea)s, poly(ether-urea)s, poly(ester-urea)s, poly(siloxane-urethane)s, poly(carbonate-urethane)s, or the like; poly(meth)acrylates, *e.g*., poly(methyl methacrylate), ethylene-(meth)acrylate copolymers, partially metal-salt-neutralized poly(meth)acrylic acids, partially aliphatichydrocarbon-saponified poly(meth)acrylic acids, or the like; poly(meth)acrylic acids; poly(vinyl acetate)s, such as ethylene-vinyl acetate copolymers; polyamides, *e.g*., nylons, aramid fibers, such as Kevlar®, poly(ester-amide) copolymers, poly(ether-amide) copolymers, or the like; polyethers, *e.g*., poly(ether-ether-ketone)s, poly(ether-ketone-ketone)s, poly(ether-ester) copolymers, poly(ether-amide) copolymers, or the like; polyimides; carbon-fiber composites; polymer-ceramic composites; polymer-metal (alloy) composites; those polymers described in U.S. Patent Nos. 4,588,580, 5,573,778, or 5,750,134, the disclosures of each of which are incorporated herein by reference; or the like; or copolymers thereof; or a combination thereof.

Particularly with respect to those embodiments comprising one or more rigid substrates, the substrate may include any polymer or polymer composite having sufficient thickness and rigidity to prevent the substrate from being folded without damaging or cracking the substrate. In addition to the foregoing polymers and polymer composites listed above, such rigid substrates may include any fiberglass, metals and/or ceramics and/or composites comprising one or more of such components with any polymer. For example, such rigid substrates may include metals such as aluminum, titanium, steel, stainless steel, as well as metallic/ceramic composites comprising such metals.

While the thickness of the substrate(s) is not particularly constrained, flexible substrates are preferably thinner than rigid substrates. In addition, the desired thickness of the substrate(s) depend on, *inter alia,* its(their) relative barrier properties for preventing, inhibiting, or diminishing misuse or abuse of, *e.g.,* the opioid agonist (*i.e.,* the better barrier the substrate material presents, *e.g*., to potential extraction solvents, the less thick it is required to be). For example, flexible substrates can advantageously have a thickness from about 0.0005 inches (13 µm) to about 0.01 inches (250 µm), preferably from about 0.001 inches (25 µm) to about 0.005 inches (130 µm), whereas rigid substrates are preferably thicker than about 0.005 inches (130 µm), preferably having a thickness from about 0.01 inches (250 µm) to about 1 inch (25 mm).

While the length and width of the substrate(s) are not particularly constrained, substrates (and particularly the portion of the substrates coated with an adhesive, if less than the entire substrate is coated) having a face with an area which approximates that of the TDD to be disposed of are preferred for booklet-type, multiple-substrate TDD disposal systems according to the invention, while it is generally only necessary that the substrates (and particularly the portion of the substrate(s) coated with an adhesive, if less than the entire substrate is coated) have a cross-sectional area sufficient to cover or contain the TDD or the active component(s) therein from misuse or abuse. That is, the desired length and width of the substrate(s) (and particularly the portion of the substrate(s) coated with an adhesive, if less than the entire substrate is coated) may depend on, *inter alia,* the relative irreversibility of the adhesion to the TDD and/or the ability to isolate or seal the TDD, or the active component(s) therein, for preventing, inhibiting, or diminishing misuse or abuse of, *e.g.,* the opioid agonist (*i.e*., the better adherent the adhesive-coated substrate is to the TDD and/or the better barrier the adhered substrate-TDD combination offers against, *e.g*., extraction by solvents, the less long and/or wide the substrate(s), or particularly the portion of the substrate(s) coated with an adhesive, if less than the entire substrate is coated, need be). In one embodiment, the cross-sectional area of the face of the substrate (and particularly the portion of the substrate coated with an adhesive, if less than the entire substrate is coated) is not more than 10% larger than the TDD to which it is to adhere, alternately not more than about 5% larger than the TDD, for example approximately the same as the TDD.

The substrate may be provided with appropriate labeling instructing the user regarding proper usage. Also, the substrate may be of a color, such as red, which contrasts with the color of a typical TDD.

The adhesive coating on the substrate(s) in the TDD disposal system according to the invention may advantageously be similar or identical to those enumerated for use in TDDs, as described previously. Additionally or alternately, the adhesive coating on the substrate(s) may include, but is(are) not limited to, epoxies, polyimides, polyamides, silicones, acrylics, polymers including repeat units having substituted or unsubstituted phenolic moieties, or the like, or a copolymer or combination thereof. Particularly preferred adhesives that may be used for coating on the substrate(s) include, but are not limited to, epoxy-based adhesives, such as those available from Durapower, and pressure sensitive adhesives, such as the acrylic-based adhesives 387-2051 and 387-3054 available from National Starch and the silicone-based adhesives 7-4102, 7-4202, 7-4302, 7-4402, 7-4502 and 7-4602 available from Dow Silicones. Without being bound by theory, it is believed that, at least on non-rigid substrates, the silicone-based adhesives have advantages, in terms of isolating the active component(s) of the TDD adhered thereto and thereby preventing, inhibiting, or diminishing its(their) misuse or abuse, at least as compared to other rubber-based adhesives, *e.g*., such as those enumerated in U.S. Patent No. 5,804,215. Additionally, it is believed that acrylic-based adhesives may also be advantageous.

Although the thickness of the adhesive coating(s) is(are) not particularly constrained, preferably the thickness is sufficient to substantially adhere the substrate on which the adhesive coating is disposed to the TDD and either to itself, to the non-adhesive-coated portion of at least one substrate, or to the second substrate, when present. For example, the adhesive coating can advantageously have a thickness from about 0.0005 inches (13 µm) to about 0.1 inches (2.5 mm), preferably from about 0.001 inches (25 µm) to about 0.01 inches (250 µm), more preferably from about 0.002 inches (50 µm) to about 0.004 inch (100 µm). In a preferred embodiment, the thickness of the adhesive coating is sufficient to substantially seal the TDD(s) within its area(s) of immobilization and isolation and to substantially irreversibly adhere the adhesive-coated substrate to itself, to the non-adhesive-coated portion of at least one substrate, or to the second substrate, when present.

Optionally but preferably, the TDD disposal system according to the invention may advantageously contain an adhesive backing material, such as a release backing layer or a layer which has been made into a release backing layer by bearing a release coating releasably disposed on the adhesive coating on the substrate, such that the backing can easily be removed from the adhesive coating, *e.g*., peeled off, when it is desired that the adhesive layer substantially adhere to the TDD, to itself, to a non-adhesive-coated portion of at least one substrate, and/or to a second substrate, when present.

In one preferred embodiment, the TDD disposal system according to the invention may advantageously contain multiple substrates, each having an adhesive coating layer, upon which is preferably releasably disposed an adhesive backing material, or a release coating, such that it can easily be removed from the adhesive coating, *e.g*., peeled off, when it is desired that the adhesive layer substantially adhere to the TDD, to itself, to a non-adhesive-coated portion of at least one substrate, and/or to a second substrate, when present. These multiple substrates can advantageously each adhere to, immobilize, isolate, and/or prevent, inhibit, or diminish the misuse or abuse of one or more TDDs.

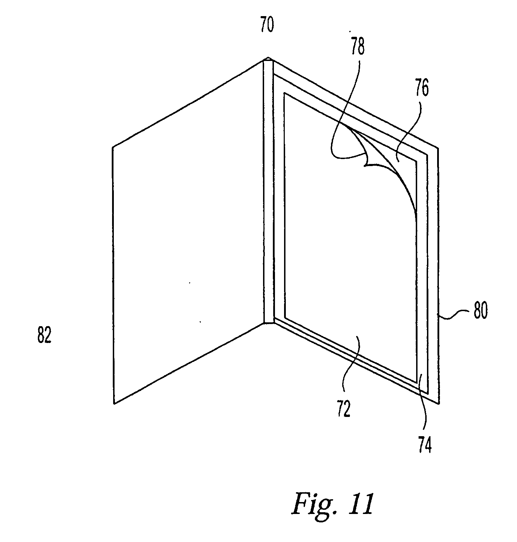

In another preferred embodiment, the TDD disposal system according to the invention comprises multiple substrates on each of which an adhesive is coated, and which optionally also comprises multiple adhesive backing materials each releasably disposed on one of the adhesive coatings on the multiple substrates, and wherein the multiple substrates are bound together in a booklet-type form. This embodiment is illustrated in Figure 11. Individual leaves, *e.g*., (72) and (74), each comprising a TDD disposal system, are bound into the form of a booklet (70). As discussed in detail above, each individual leaf is made of at least one substrate coated with adhesive, *e.g*., (76), the leaf optionally comprising the following component(s): a monomer, and optionally at least one comonomer; an initiator, and optionally at least one co-initiator; at least one crosslinking agent; at least one opioid agonist deactivating agent, *e.g*., an opioid antagonist; and mixtures thereof. In the embodiment illustrated in Figure 11, a protective adhesive backing material, such as the release backing layer (78) illustrated in the figure, is removed from each leaf and disposed of prior to using that leaf to dispose of at least one TDD. The booklet, here having covers (80) and (82), is disposed of in an appropriate manner when spent, *e.g*., each leaf has been utilized for the disposal of TDDs. If desired, appropriate text providing TDD disposal instructions may be provided on the disposal system, such as a substrate release layer.

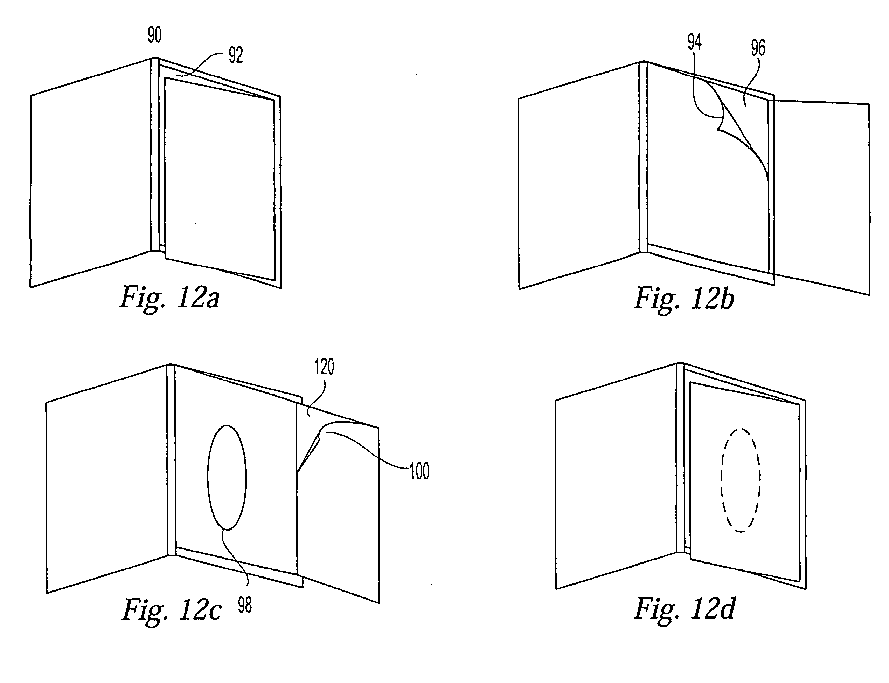

In a further preferred embodiment, the TDD disposal system according to the invention comprises multiple bi-folded substrates on each of which an adhesive is coated, and which optionally also comprises multiple adhesive backing materials each releasably disposed on one of the adhesive coatings on the multiple substrates, and wherein the multiple substrates are bound together in a booklet-type form. This embodiment is illustrated in Figure 12A. Individual bi-folded leaves, only one of which (92) is shown for simplicity, each comprising a TDD disposal system, are bound into the form of a booklet (90). As discussed in detail above, each individual bi-folded leaf is made of at least one substrate coated with adhesive, the leaf optionally comprising the following component(s): a monomer, and optionally at least one comonomer; an initiator, and optionally at least one co-initiator; at least one crosslinking agent; at least one opioid agonist deactivating agent; and mixtures thereof.

The multiple bi-folded leaf TDD disposal system booklet is used to dispose of TDDs as follows. Figure 12B illustrates the removal of a portion of a protective adhesive backing material, such as the release backing layer (94) illustrated in the figure, from a leaf so that at least one TDD, *e.g*., a spent TDD removed from a patient, can be adhered to the exposed adhesive coating (96) of the leaf. Preferably, the adhesive coated surface of the TDD is contacted with the adhesive coating of the leaf. The removed release backing layer is appropriately disposed of after it is removed. Figure 12C illustrates the TDD (98) in place on the adhesive of the leaf and the removal of another portion of the protective adhesive backing material, such as the release backing layer (100) illustrated in the figure, from the leaf. The removed release backing layer is appropriately disposed of after it is removed. Figure 12D illustrates the portion of the bi-folded leaf with exposed adhesive (102) being folded over the TDD so as to substantially isolate the TDD. The booklet is disposed of in an appropriate manner when spent, *e.g*., each leaf has been utilized for the disposal of TDDs. This style of disposal system is easily adapted to integrate into a preferred packaging presentation while at the same time satisfying the need to reduce abuse potential. If desired, appropriate text providing TDD disposal instructions may be provided on the disposal system, such as on a substrate or a release layer.

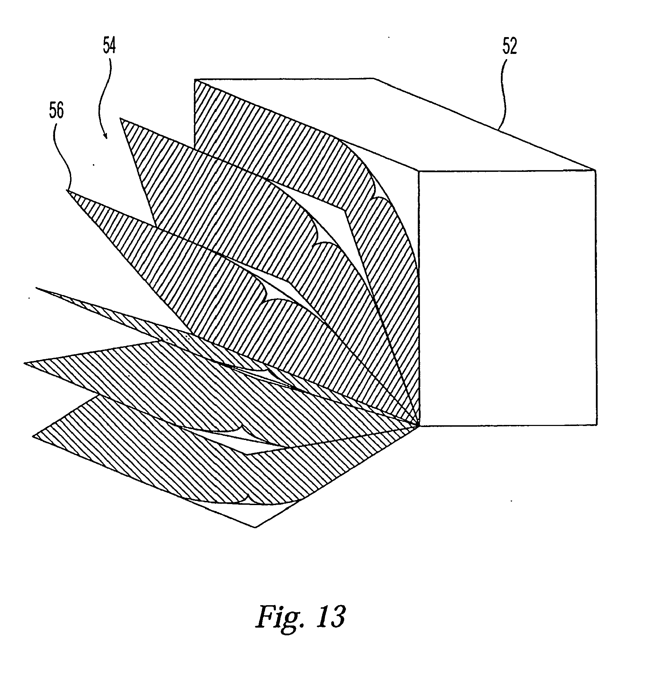

As illustrated in Figure 13, in certain embodiments, the present invention comprises a kit including a box 52 for enclosing one or more patches and a TDD disposal system in the form of a booklet 54 comprising multiple substrates 56.

In a further preferred embodiment, the TDD disposal system according to the invention is one or more of the following: substantially solvent impermeable; substantially not solvent swellable; substantially tear-resistant; substantially cut-resistant; substantially sealed, with respect to the area(s) of immobilization and isolation of the TDD(s) contained therein, once the TDD(s) is(are) contained therein; substantially irreversibly adhered, once the adhesive-coated substrate is substantially adhered to itself, to a non-adhesive-coated portion of at least one substrate, *e.g*., by folding, or to the second substrate, when present; substantially a barrier to extraction of an active agent, *e.g*., the opioid agonist, contained in the TDD; or the like; or any combination thereof.

Optionally but preferably, the TDD disposal system according to the invention includes one or more (alternately, two or more) of the following components: monomer (and optionally comonomers) to be polymerized; an initiator (and optionally coinitiators) to initiate the polymerization, preferably through activation by energy, *e.g*., such as ultraviolet, UV-VIS, or visible light and/or thermal energy (or infrared radiation); at least one crosslinking agent to crosslink the (co)polymer; at least one opioid agonist deactivating agent, such as an opioid antagonist, to counteract, inhibit, diminish, or halt the euphoric effects of the opioid agonist(s) contained in the TDD, in case an attempt is made to misuse or abuse the opioid agonist(s) contained in the TDD or to render the opioid agonist unavailable through deactivation, biounavailability, physical unavailability, loss of appeal of the active agent to the abuser, or the like, or a combination thereof; at least one opioid agonist deactivating agent to chemically alter, degrade, and/or deactivate the opioid agonist(s) contained in the TDD, so that, in case of misuse or abuse, the euphoric effects of the opioid agonist(s) contained in the TDD are at least temporarily (and preferably permanently, in their altered, degraded, and/or deactivated form) inhibited, diminished, or halted *in vivo*; or the like; or a combination thereof. In one embodiment, the TDD disposal system according to the invention can include a non-µ-opioid deactivating agent.

If present, these optional components may advantageously be included in and/or on any portion of the TDD disposal system according to the invention, so that these optional components may come into contact with the TDD, once the TDD is contained within the TDD disposal system according to the invention. In one preferred embodiment, these optional components are dissolved or dispersed within the adhesive coating on at least one substrate. Additionally or alternately, these optional components may be present in and/or on the second substrate, when present.

In one embodiment, at least one of the optional components is present in a first region of the adhesive coating, on at least one substrate and/or on the second substrate, when present, while at least one other of the optional components is present in a second region of the adhesive coating, on at least one substrate and/or on the second substrate, when present. In another embodiment, all of the optional components that are present are located in the same region of the adhesive coating, on at least one substrate and/or on the second substrate, when present.

The monomer and/or comonomer(s), if present, that may be included in the TDD disposal system according to the invention can advantageously include any monomer and/or comonomer(s) that can be used to make, *e.g.,* the adhesive (coating) layer of the TDD disposal system and/or of the TDD, the rate-controlling membrane material of the TDD, the material of at least one of the substrates, or the like, or a blend or copolymer thereof. Exemplary monomers/comonomers include, but are not limited to, diisocyanates, diols, diacids (preferably carboxylic), diesters, diamines, epoxides, diepoxides, cyanoacrylates, (meth)acrylic acid, monovalent (meth)acrylate metal salts, organic (meth)acrylates, vinyl acetate, poly(vinyl alcohol) precursors, cellulosic monomers/oligomers (*e.g*., cellulose acetate, cellulose propionate, cellulose butyrate, cellulose acetate-propionate, cellulose acetate-butyrate, cellulose propionate-butyrate, cellulose nitrate, methyl cellulose, ethyl cellulose, carboxymethyl cellulose, carboxyethyl cellulose, cellulose salts, and combinations or copolymers thereof, which may optionally be modified in some way, such as partially or fully esterified, partially or fully nitrated, partially or fully regenerated, partially or fully etherified, partially or fully acidified, partially or fully acid-neutralized, or the like, or combinations thereof), room-temperature stable α-olefins, silicon-containing monomers (*e.g*., organosilanes, siloxanes, or the like, or a combination thereof), phosphazenes, or the like, or any combination thereof.

The initiator and/or co-initiator(s), if present, that may be included in the TDD disposal system according to the invention can advantageously include any initiators/co-initiators known in the art to initiate the (co)polymerization of the monomer (and/or comonomer(s)) present in the TDD disposal system according to the invention and preferably specifically selected for use with the particular monomer/comonomer(s). One of ordinary skill in the art would be able to make such specific selection through limited routine experimentation, if required. Examples of initiators/co-initiators include, but are not limited to, monoamines (*e.g*., for initiating epoxides), free-radical-forming compounds (*e.g*., peroxides, bis-azo compounds, or the like, such as for initiating α-olefins; (meth)acrylic acids, or metal salts thereof; organic (meth)acrylates; or the like), particularly those compounds that are UV-, photo-, or thermally-activated, or the like, or any combination thereof.

Many types of free-radical initiators are suitable for initiating crosslinking, *e.g*., azo and diazo compounds, such as azo-bis-isobutyronitrile ("AIBN"), organic peroxides, hydroperoxides, persulfates and hydropersulfates, such as benzoyl peroxide, inorganic peroxides and persulfates, such as the peroxide-redox systems, carbon-carbon initiators, such as hexasubstituted ethanes, and photoinitiators; numerous examples are known in the art. (*See* Sanchez et al., "Initiators (Free-Radical)" in Kirk-Othmer Encyc. of Chem. Technol., 4th Ed., John Wiley & Sons, New York, 1995, Vol. 14, pp. 431-460, the disclosure of which is incorporated herein by reference.) Suitable anionic initiators for initiating crosslinking are known in the art and include aromatic radical anions, such as sodium naphthalene; alkyl lithium compounds, such as t-butyl lithium; fluorenyl carbanions; 1,1-diphenylmethylcarbanions; cumyl potassium; and those described by Quirk et al., "Initiators (Anionic)" in Kirk-Othmer Encyc. of Chem. Technol., 4th Ed., John Wiley & Sons, New York, 1995, Vol. 14, pp. 461-476, the disclosure of which is incorporated herein by reference. Suitable cationic initiators for initiating crosslinking are also known in the art and include protic acids, cation donor (initiator)/Friedel-Crafts acid (co-initiator) systems, stable cation salts, and those described by Faust, "Initiators (Cationic)" in Kirk-Othmer Encyc. of Chem. Technol., 4th Ed., John Wiley & Sons, New York, 1995, Vol. 14, pp. 476-482, the disclosure of which is incorporated herein by reference.

The free-radical, anionic, or cationic initiator may undergo decomposition by any known means, *e.g*., thermally or photolytically, when this is required to initiate polymerization and/or crosslinking. In particular, photoinitiators, such as those described in McGinniss, "Radiation Curing" in Kirk-Othmer Encyc. of Chem. Technol., 4th Ed., John Wiley & Sons, New York, 1996, Vol. 20, pp. 848-850, the disclosure of which is incorporated herein by reference, are well-known in the art for initiating crosslinking.

For example, free-radical-forming compounds can include, but are in no way limited to, dicumyl peroxide (*e.g*., commercially available as PEROXIMON DC 400KEP® from Elf Atochem N.A., and as ESPERAL 115RG® from Witco); 1,1-di(t-butylperoxy)-3,3,5-trimethylcyclohexane (*e.g*., commercially available as VAROX 231XL® from R.T. Vanderbilt, and as LUPERCO 231KE® from Elf Atochem N.A.); α,α'-bis(t-butylperoxy)-diisopropylbenzene (*e.g*., commercially available as RETILOX F40KEP® from Elf Atochem N.A.); 2,5-dimethyl-2,5-di(t-butylperoxy)hexane (*e.g*., commercially available as VAROX DBPH-50® from R.T. Vanderbilt, and as LUPERCO 101-XL® from Elf Atochem N.A.); di(t-butyl) peroxide (*e.g*., commercially available from Witco); 2,5-dimethyl-2,5-di-(tert-butylperoxy)-3-hexyne; tert-butyl cumyl peroxide; n-butyl-(4,4-di-tert-butylperoxy)-valerate; tert-amyl peroxybenzoate; 4,4-azobis-(4-cyanovaleric acid); 1,1'-azobis-(cyclohexanecarbonitrile); 2,2'-azobis-isobutyronitrile; benzoyl peroxide; 2,2-bis-(tert-butylperoxy)-butane; 1,1-bis-(tert-butylperoxy)-cyclohexane; 2,5-bis-(tert-butylperoxy)-2,5-dimethylhexane; bis-[1-(tert-butylperoxy)-1-methylethyl]-benzene; tert-butyl hydroperoxide; tert-butyl peracetate; tert-butylperoxy isopropyl carbonate; tert-butyl peroxide; tert-butyl peroxybenzoate; cumene hydroperoxide; cyclohexanone hydroperoxide; lauroyl hydroperoxide; stearoyl hydroperoxide; 2,4-pentanedione peroxide; peracetic acid; or the like; or any combination thereof.

Polymerization and/or crosslinking may, of course, also be effected by high energy ionizing radiation sources. For example, crosslinking can be brought about by exposing a combination of a polymer(s) and monomer(s), especially monomer(s) with an average functionality of greater than 2, to electron beams, ultraviolet radiation, usually in the presence of a photoinitiator, and high energy ionizing radiation sources, such as γ-radiation from a ⁶⁰Co or ¹³⁷Cs source, a-particles, β-particles, fast neutrons and x-rays. Each of these techniques can cause the generation of free-radicals and/or ions that, in turn, initiate polymerization and/or crosslinking. (*See* Sanchez et al., "Initiators (Free-Radical)," at 454-457; Sheppard et al., "Initiators," in Kirk-Othmer Encyc. of Chem. Technol., 3rd Ed., John Wiley & Sons, New York, 1981, Vol. 13, pp. 367-370, the disclosure of which is incorporated herein by reference).

At least the three following radiation crosslinking or grafting methods are well-known in the art: (1) the "pre-irradiation" method, in which the polymer is irradiated before interacting with the monomer(s), (2) the "mutual radiation" method, in which the polymer and the monomer(s) are in contact while irradiation occurs, and (3) the "peroxide" method, in which the polymer is irradiated in the presence of air or oxygen before interacting with the monomer(s). (*See* Stannett et al., "Polymerization by High-Energy Radiation" in Comprehensive Polymer Science, Pergamon Press, Oxford, 1989, Vol. 4, Eastmond et al., Eds., p. 327-334, the disclosure of which is incorporated herein by reference).

Thus, the free-radical initiating means for crosslinking, and/or polymerization, which can function by a thermal and/or photolytic dissociation, can be an initiator selected from the group consisting of azo compounds, diazo compounds, organic peroxides, organic hydroperoxides, organic persulfates, organic hydropersulfates, inorganic peroxides, inorganic persulfates, peroxide-redox systems, carbon-carbon initiators, photoinitiators, and mixtures thereof.

The crosslinking agent(s), if present, that may be included in the TDD disposal system according to the invention can advantageously include any crosslinking agent known in the art to chemically or physically crosslink the monomer (and/or comonomer(s)) present, or the oligomers and/or (co)polymers subsequently formed therefrom, in the TDD disposal system according to the invention and are preferably selected for the particular (co)monomers/oligomers/(co)polymers used. One of ordinary skill in the art would be able to make such selection through limited routine experimentation, if required.

Crosslinking agents preferably have either (a) functional group(s) capable of reacting with multiple functional groups on or pendent to an already-formed oligomer or (co)polymer or (b) polymerizable group(s) capable of reacting with multiple propagating oligomer or (co)polymer chain ends, thus forming interchain crosslinks within an oligomer or (co)polymer. Examples of crosslinking agents include, but are not limited to, compounds having, on average, one or more of the following: more than two isocyanate functional groups, more than two hydroxyl functional groups, more than two ester functional groups (e.g., for trans- or inter- esterification reactions), more than two (preferably carboxylic) acid functional groups, more than two amine functional groups (i.e., each having at least one reactive site), one or more epoxide functional groups, more than one polymerizable unsaturated group, more than two monovalent carboxylate metal salt groups, or the like, or more than two of a combination of the functional groups listed above.

Crosslinking agents may also react with a previously unreactive site on an already-formed oligomer or (co)polymer to activate it. Then, that active site can itself react with a reactive or previously unreactive site on another already-formed oligomer or (co)polymer chain. Alternatively, that reactive site can react with other compounds present in the TDD disposal system according to the invention, e.g., such as monomers/comonomers mentioned above, that eventually react with a reactive or previously unreactive site on another already-formed oligomer or (co)polymer chain.

There is at least one active agent deactivating agent which may be included in the TDD disposal system according to the invention. For example, any opioid agonist deactivating agent known in the art to chemically alter, degrade, and/or deactivate the opioid agonist(s) contained in the TDD, so that, in case of misuse or abuse, the euphoric effects of the opioid agonist(s) contained in the TDD are at least temporarily (and preferably permanently, in their altered, degraded, and/or deactivated form) inhibited, diminished, or halted in vivo, may be included. The opioid agonist deactivating agent, if present, is preferably specifically selected for use with the particular opioid agonist(s) contained in the TDD, although certain opioid agonist deactivating agents cause the chemical alteration, degradation and/or deactivation of many common opioid agonists. One of ordinary skill in the art would be able to make such specific selection through limited routine experimentation, if required.

Other examples of active agent deactivating agents include, but are in no way limited to, strongly oxidizing compounds and strongly reactive free-radical-forming compounds, e.g., such as those peroxide, peracid, per(metal oxide), per(non-metal oxide), and azo compounds listed as initiators (and/or co-initiators) above, or a combination thereof. Additionally or alternately, the deactivating agents may include, but are not limited to, hydrogen peroxide; per(non-metal oxide) acids, such as periodic acid; or the like; or a combination thereof.

Another example of an active agent deactivating agent which may be included is an active agent deactivating agent, such as an opioid antagonist. There is at least one opioid agonist deactivating agent which may be included in the TDD disposal system according to the invention can advantageously include any opioid agonist deactivating agent known in the art, such as any opioid antagonist, to counteract, inhibit, diminish, or halt the euphoric effects in vivo of the opioid agonist(s) contained in the TDD, in case an attempt is made to misuse or abuse the opioid agonist(s) contained in the TDD. The opioid agonist deactivating agent, if present, is preferably specifically selected for use with the particular opioid agonist(s) contained in the TDD, although certain opioid antagonists antagonize the euphoric effects of many common opioid agonists. One of ordinary skill in the art would be able to make such specific selection through limited routine experimentation, if required.

Opioid agonist deactivating agents can advantageously render the active agent unavailable through inactivating, such as for example chemical inactivation or alteration of the receptor binding site of the opioid agonist; mechanical inactivation or alteration of the receptor binding site of the opioid agonist; biounavailability; physical unavailability; loss of appeal of the opioid agonist to the abuser, e.g., a deactivating agent which creates an intolerably bad taste or an intolerable reaction, such as extreme nausea or the like; or something similar thereto; or any combination thereof.

For example, for an opioid agonist in a transdermal patch or other delivery device to be disposed of by placing it in the present TDD disposal system, the deactivating agent could be a chemical or denaturing agent that would alter residual opioid molecules in the dosage form and make them inactive. The deactivating agent could alternately or additionally be an opioid receptor that would bind the residual opioid into an insoluble ligand-receptor complex. The deactivating agent could alternately or additionally be an opioid receptor antagonist, preferably with greater specificity and/or affinity for the receptor than the opioid, which would be isolated or delivered with the residual opioid upon misuse/abuse to compete with the residual opioid for the opioid receptor, thereby defeating the purpose of misusing/abusing the opioid. This would render the residual opioid useless in vivo. The deactivating agent could alternately or additionally physically sequester the residual opioid agonist, e.g., in an impermeable microsphere or in a permanently bound matrix. Similarly, the deactivating agent could alternately or additionally be a non-opioid with distressing or dysphoric properties if absorbed that made misuse/abuse unappealing.

Examples of deactivating agents include, but are in no way limited to, the rat or human µ-opioid receptor; opioid-neutralizing antibodies; opioid/narcotic antagonists such as naloxone, naltrexone, nalmefene, cyclazacine, cyclazocine, nalorphine, nalbuphine, levallorphan, butorphanol, pentazocine, or the like, or a combination thereof; dysphoric or irritating agents such as scopolamine, ketamine, atropine, mustard oils, or the like, or a combination thereof; or the like; or any combinations thereof.

In one embodiment, the TDD disposal system can include a non-µ-opioid deactivating agent, which can include, but are in no way limited to, the rat or human κ-opioid receptor; the rat or human δ-opioid receptor; naltrindole; norbinaltorphimine; J-113397 *(i.e.,* as disclosed in Ozaki et al. (2000) Eur. J. Pharmacol., 402:45); JTC-801 (*i.e*., as disclosed in Yamada et al. (2002) Br. J. Pharmacol., 135:323); [Nphe1,Arg14,Lys15]nociceptin-NH₂ (as disclosed in Calo et al. (2002) Br. J. Pharmacol., 136:303); and combinations thereof. Other suitable non-µ-opioid deactivating agents should be known to those skilled in the art.

It should be noted that certain components may be advantageously selected to perform multiple roles in the transdermal delivery disposal systems of the present invention. For example, by incorporating a peroxide into the system, that peroxide may act as a deactivating agent for the pharmaceutical compound(s) in the TDD, such as an opioid agonist, and also act as the initiator and/or co-initiator(s), if present, to initiate the (co)polymerization of the monomer (and/or comonomer(s)) present in the TDD disposal system.

When present in the TDD disposal system according to the invention, the total amount of monomer (including any comonomers) present can be from about 0.01% to about 15%, preferably from about 0.1% to about 10%, alternately from about 0.1% to about 5%, each by weight based on the total weight of the layer in and/or on which the monomer is present.

When present in the TDD disposal system according to the invention, the total amount of initiator (including any co-initiators) present can be from about 0.001% to about 5%, preferably from about 0.01% to about 3%, alternately from about 0.1% to about 2%, each by weight based on the total weight of the layer in and/or on which the initiator is present.

When present in the TDD disposal system according to the invention, the total amount of crosslinking agent present can be from about 0.1 % to about 20%, preferably from about 0.5% to about 10%, alternately from about 1% to about 5%, each by weight based on the total weight of the layer in and/or on which the crosslinking agent is present.

When present in the TDD disposal system according to the invention, the total amount of deactivating agent present can be from about 0.001% to about 25%, preferably from about 0.0 1 % to about 15%, alternately from about 0.1 % to about 5%, each by weight based on the total weight of the layer in and/or on which the deactivating agent is present.

When present in the TDD disposal system according to the invention, the total amount of deactivating agent present can be from about 0.001 % to about 15%, preferably from about 0.01% to about 10%, alternately from about 0.1% to about 5%, each by weight based on the total weight of the layer in and/or on which the deactivating agent is present.

Additionally or alternately, certain aspects of the TDD disposal system according to the invention, or any of its components, are described in co-pending PCT Patent Application No. PCT/US02/12920 designating the United States, filed April 23, 2002, and in U.S. Provisional Patent Application Nos. 60/285,862 and 60/292,601, filed April 23, 2001 and May 22, 2001, respectively, and to both of which the PCT application claims priority. Accordingly, the disclosures of each of the PCT and provisional applications mentioned directly above are hereby incorporated herein by reference in their entirety.

Another aspect of the invention relates to a method for disposing of a TDD and/or for preventing the misuse or abuse of a component of a TDD (e.g., an opioid agonist). The method includes providing a TDD disposal system according to the invention, containing at least one substrate on which an adhesive layer is coated. If there is only one substrate on which the adhesive is coated, it can preferably be flexible, so as to be capable of folding over onto itself in order to immobilize and isolate a TDD placed thereon. Alternately, if there is only one substrate on which an adhesive is coated, a second complementary substrate may be present, which second substrate is, preferably, capable of being irreversibly adhered to at least one adhesive-coated substrate. In this alternate embodiment, the adhesive-coated substrate and the second complementary substrate may be flexible or rigid, as desired. Advantageously, the substrates and adhesives can include those described previously.

Optionally but preferably, an adhesive backing material, or a release coating releasably disposed on the adhesive coating on the substrate, is also provided, such that it can easily be removed from the adhesive coating, e.g., peeled off, when it is desired that the adhesive layer substantially adhere to itself, to a non-adhesive-coated portion of at least one substrate, or to a second substrate, when present.

The method further includes placing a TDD on a region of the adhesive coating and substantially covering or containing the TDD within at least that region to immobilize and isolate the TDD therein, either by folding the substrate, such that the adhesive coating substantially adheres to itself or to a non-adhesive-coated portion of at least one substrate, or by contacting the adhesive coating on the substrate with a second substrate, such that the adhesive coating substantially adheres to the second substrate. Additionally or alternately, the method may further include substantially irreversibly adhering the TDD to at least one region of the adhesive coating or of at least one substrate, in order to immobilize and isolate the TDD thereon, and then forming an immobilization and isolation region within which the TDD can be contained.

Optionally but preferably, the method can also include providing one or more of the following components within the TDD disposal system according to the invention: monomer (and optionally comonomers) to be polymerized into a (co)polymer; an initiator (and optionally co-initiators) to initiate the polymerization of the (co)monomers, preferably through activation by energy, e.g., such as ultraviolet, UV-VIS, or visible light and/or thermal energy (or infrared radiation); at least one crosslinking agent to crosslink the (co)polymer; at least one opioid agonist deactivating agent to counteract, inhibit, diminish, or halt the euphoric effects of the opioid agonist(s) contained in the TDD, in case an attempt is made to misuse or abuse the opioid agonist(s) contained in the TDD or to render the opioid agonist unavailable through inactivation, biounavailability, physical unavailability, loss of appeal of the active agent to the abuser, or the like, or a combination thereof; at least one opioid agonist deactivating agent to chemically alter, degrade, and/or deactivate the opioid agonist(s) contained in the TDD, so that, in case of misuse or abuse, the euphoric effects of the opioid agonist(s) contained in the TDD are at least temporarily (and preferably permanently, in their altered, degraded, and/or deactivated form) inhibited, diminished, or halted in vivo; or the like; or a combination thereof.

If present, these optional components may advantageously be provided in and/or on any portion of the TDD disposal system according to the invention, so that these optional components may come into contact with the TDD, once the TDD is contained within the TDD disposal system according to the invention. In one preferred embodiment, these optional components are dissolved or dispersed within the adhesive coating on at least one substrate. In another preferred embodiment, these optional components are dissolved or dispersed within at least one substrate. Additionally or alternately, these optional components may be provided in and/or on the second substrate, when present.

In one preferred embodiment, the method includes providing multiple substrates, each having an adhesive coating layer, upon which is preferably releasably disposed an adhesive backing material, or a release coating, such that it can easily be removed from the adhesive coating, e.g., peeled off, when it is desired that the adhesive layer substantially adhere to the TDD, to itself, to a non-adhesive-coated portion of at least one substrate, and/or to a second substrate, when present. These multiple substrates can advantageously each adhere to, immobilize, isolate, and/or prevent, inhibit, or diminish the misuse or abuse of one or more TDDs.

In another preferred embodiment, the method includes providing a TDD disposal system comprising multiple substrates on each of which an adhesive is coated, and which optionally also comprises multiple adhesive backing materials each releasably disposed on one of the adhesive coatings on the multiple substrates, and wherein the multiple substrates are bound together in a booklet-type form. The multiple substrates of this booklet can each conveniently and advantageously each adhere to, immobilize, isolate, and/or prevent, inhibit, or diminish the misuse or abuse of one or more TDDs.

### EXAMPLES

The following examples serve to illustrate, rather than limit, the scope of the present invention.

### Example 1

In Example 1, a transdermal delivery device according to Figure 2B was loaded with buprenorphine (BUP). The specific BUP TDD used for Example 1 was similar in composition to those sold in Europe by Gruenenthal of Switzerland under the tradename TRANSTEC, but it contained only a 20 mg total BUP dosage (and therefore had a different surface area than the commercially available TDDs). The BUP TDD was then immobilized and isolated with a TDD disposal system according to Figure 1B. Then, the amount of BUP extractable from the TDD (and thus potentially available for abuse), in some cases contained with the TDD disposal system, was determined through extraction using the following solvents: distilled water, tap water, an 0.026M aqueous baking soda solution, 5% vinegar, acetone, methanol, ethanol, ethyl acetate, or ethyl ether. The extractions were conducted under the following extraction conditions: after 5 minutes, 60 minutes, and 120 minutes in the extraction solvent at room temperature, about 25°C. For each extraction solvent, the TDD was present in the following states of isolation: (1) without folding the TDD substrate ("open face"), (2) with the substrate folded upon and adhered to itself ("folded over"), (3) with the substrate folded upon and adhered to itself and then cut into between about 10 and about 50 pieces ("folded and cut"), and, (4) without folding, while adhering the TDD substrate to the adhesive of a TDD disposal system (device adhered adhesive side down) ("adhesive film covered").

The TDD disposal system used throughout consisted of a film of poly(ethylene terephthalate)/poly(ethylene-co-vinyl acetate) about 46 µm thick obtained from the 3M Company (#9733) coated with a silicone pressure sensitive adhesive obtained from Dow Silicones (#7-4302) about 220 µm thick. To protect the adhesive, a release coated poly(ethylene terephthalate) film about 74 µm thick obtained from the 3M Company (#1022) was applied thereto. The release coated film was removed shortly before the TDD disposal system was adhered to the BUP TDD.

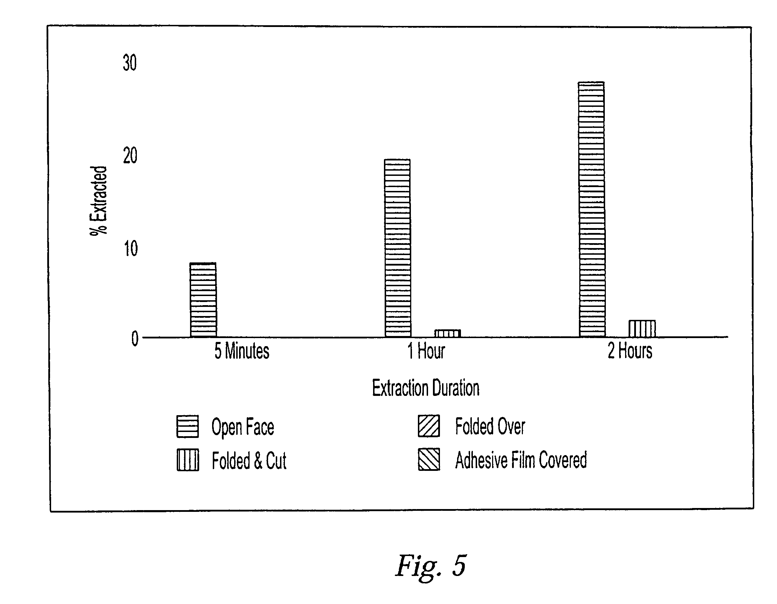

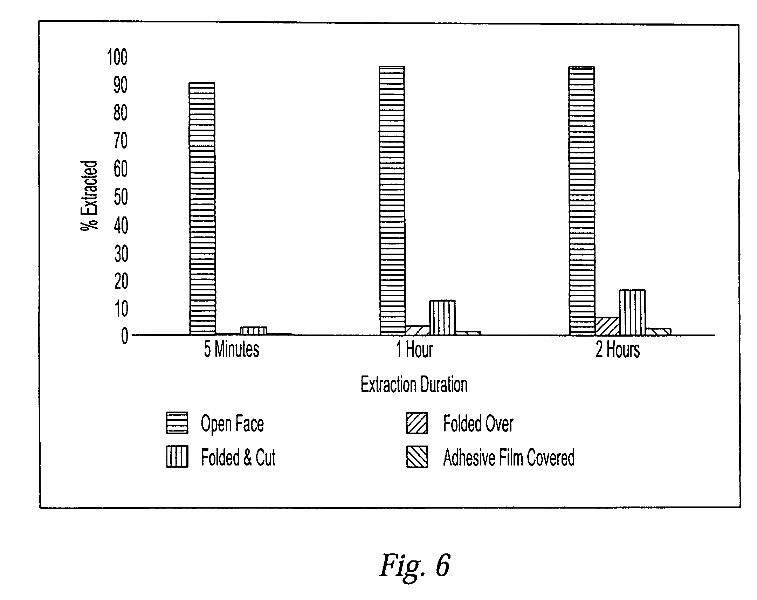

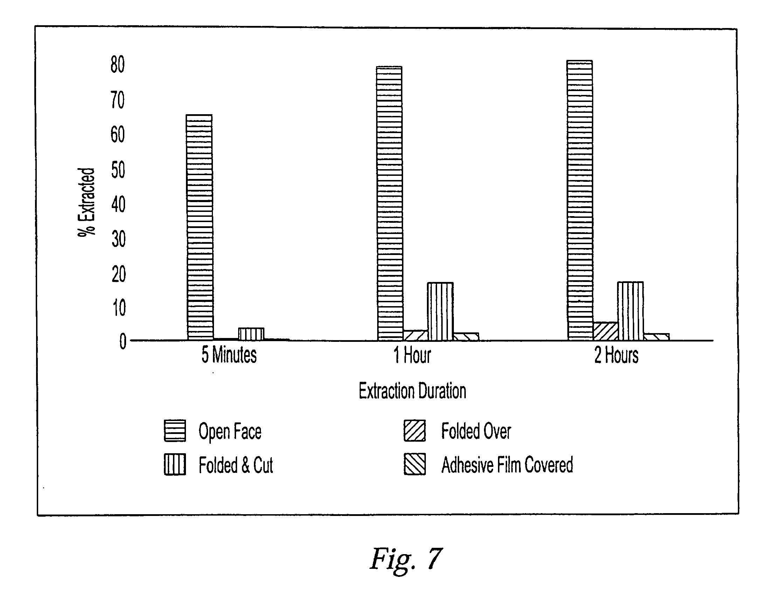

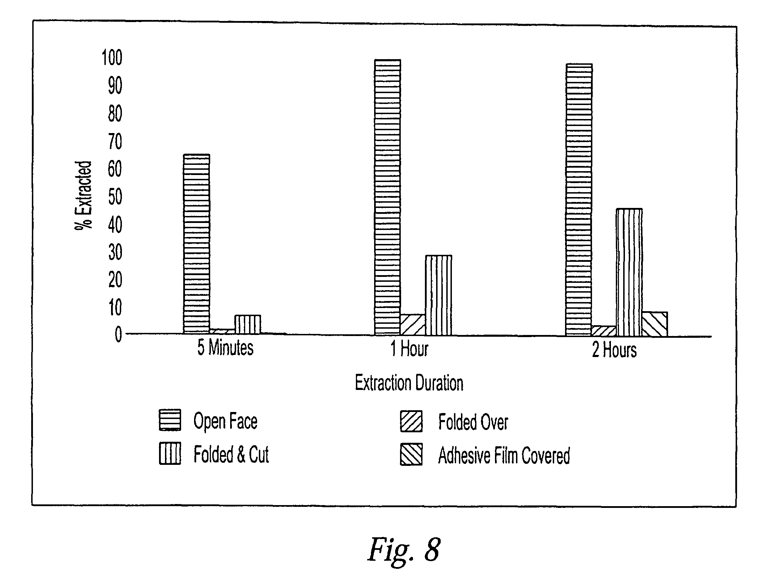

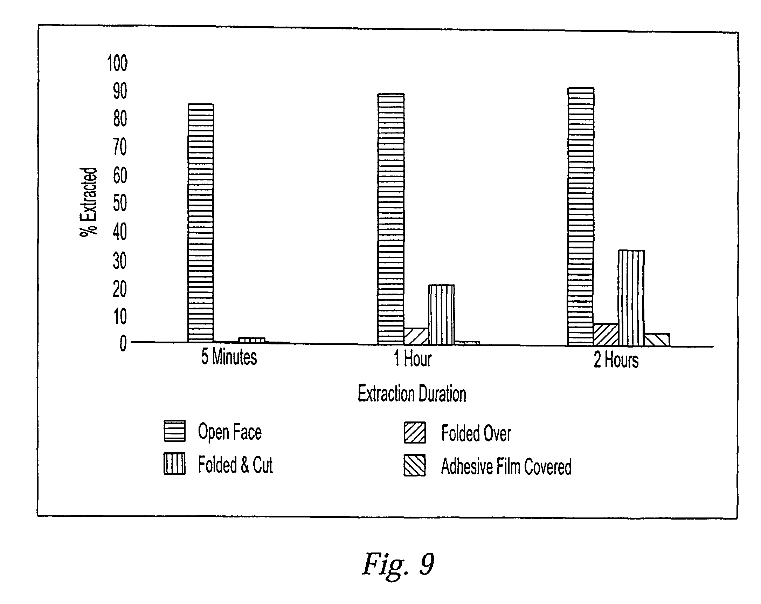

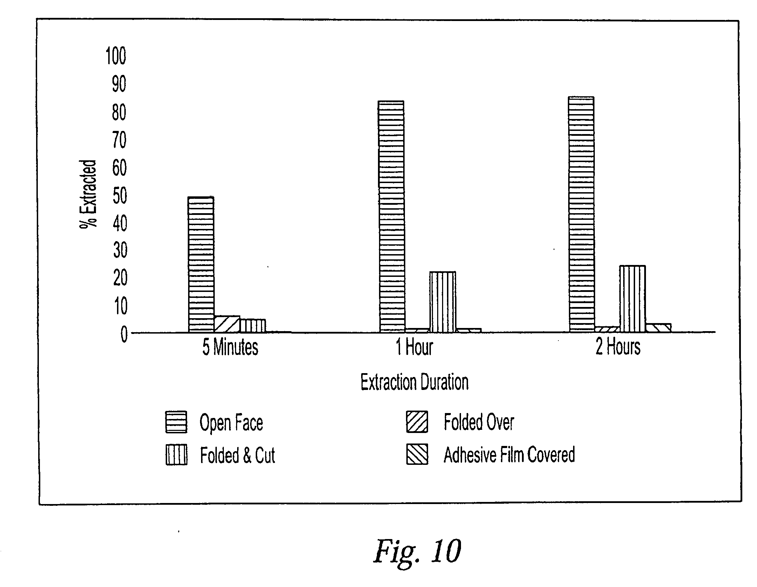

Each of Figures 5-10 shows the percentage of BUP extractable by distilled water, ethyl acetate, ethanol, ethyl ether, acetone, and methanol, respectively, from the TDD present in each of the above states of isolation. Table 1 below, from which Figures 5-10 were constructed, shows the data for each extraction example.

**Table 1**

| % OF BUPRENORPHINE SOLVENT-EXTRACTED AFTER | | | | | | | | | | | | |
|---|---|---|---|---|---|---|---|---|---|---|---|---|
| SOLVENT | 5 minutes | | | | 1 hour | | | | 2 hours | | | |
| | Open Face | Folded Face | Folded & Cut | Adhesive Film Covered | Open Face | Folded Face | Folded & Cut | Adhesive Film Covered | Open Face | Folded Face | Folded & Cut | Adhesi Film Coven |
| Distilled Water (6.6 pH) | 8.1 | 0 | 0 | 0 | 19.3 | 0 | 0.8 | 0 | 27.7 | 0 | 2.0 | 0 |
| Tap Water (7.4 pH) | 7.3 | 0 | 0 | 0 | 20.9 | 0 | 0 | 0 | 30.5 | 0 | 3.9 | 0 |
| 0.026M Baking Soda Solution (8.2 pH) | 1.1 | 0 | 0 | 0 | 0.9 | 0 | 0 | 0 | 0.9 | 0 | 0 | 0 |
| 5% Vinegar (2.4 pH) | 5.9 | 0 | 0.2 | 0 | 18.5 | 0 | 1.8 | 0 | 27.1 | 0 | 2.2 | 0 |
| Acetone | 83.4 | 0.4 | 1.6 | 0.3 | 88.5 | 5.9 | 21.7 | 1.1 | 91.5 | 8.6 | 34.4 | 4.9 |
| Methanol | 41.9 | 6.2 | 4.9 | 0 | 83.4 | 1.5 | 21.5 | 0.8 | 84.9 | 2.0 | 23.4 | 3.1 |
| Ethanol | 64.9 | 0.2 | 3.4 | 0 | 78.3 | 2.7 | 16.7 | 1.9 | 79.7 | 4.9 | 16.8 | 1.5 |
| Ethyl Acetate | 89.6 | 0.4 | 3.0 | 0.2 | 96.3 | 3.5 | 12.9 | 1.6 | 96.2 | 6.8 | 16.8 | 2.6 |
| Ethyl Ether | 64.9 | 1.9 | 7.2 | 0.2 | 100 | 7.8 | 29.1 | - | 98.5 | 4.1 | 46.7 | 9.3 |

### Prophetic Example 2

For prophetic Example 2, a transdermal delivery device according to Figure 2B is loaded with BUP, as in Exmaple 1. The BUP TDD is then immobilized and isolated with a TDD disposal system according to Figure 1C. Then, the amount of BUP extractable from the TDD (and thus available for abuse), in some cases contained with the TDD disposal system, is determined through extraction by the following solvents: distilled water, tap water, an 0.026M aqueous baking soda solution, 5% vinegar, acetone, methanol, ethanol, ethyl acetate, and/or ethyl ether. The extractions are conducted under the following extraction conditions: after 5 minutes, 60 minutes, and 120 minutes in the extraction solvent at room temperature, about 25°C, and at reflux *(i.e.,* at approximately the boiling temperature of the extraction solvent). For each extraction solvent, the TDD is present in the following states of isolation: (1) without adhering the first substrate *(i.e.,* that is already adhered to the TDD) to a second substrate (device adhered adhesive side up) ("open face"), (2) with the TDD adhered between the first and second substrates ("sandwiched"), (3) with the TDD adhered between the first and second substrates and then cut or broken into between about 10 and about 50 pieces ("folded and cut"), and (4) without adhering the first substrate *(i.e.,* that is already adhered to the TDD) to a second substrate (device adhered adhesive side down) ("adhesive film covered").

The TDD disposal system for use in this example consists of a substrate film of poly(ethylene terephthalate)/poly(ethylene-co-vinyl acetate) about 46 µm thick obtained from the 3M Company (#9733) coated with an adhesive mixture. The adhesive mixture includes about 95% by weight of an acrylic-based adhesive that is obtained from Solutia (#2464), in combination with about 5% benzoyl peroxide (based on the solids content of the adhesive mixture). The adhesive mixture coating is between about 150-350 µm thick. To protect the adhesive mixture, a release coated poly(ethylene terephthalate) film about 50-100 µm thick from the 3M Company (#1022) is applied thereto. The release coated film is removed before the TDD disposal system is adhered to the active agent-containing TDD.

The present invention is not to be limited in scope by the specific embodiments described herein. Indeed, various modifications of the invention in addition to those described herein will become apparent to those skilled in the art from the foregoing description and the accompanying figures. Such modifications are intended to fall within the scope of the appended claims.

All patents, applications, publications, test methods, literature, and other materials cited are hereby incorporated herein by reference.

Further embodiments of this invention are the following:
1. A transdermal delivery device disposal system comprising:
   at least a first substrate having an adhesive coating on one face thereof; and
   one or more of the following components present at least in or on the adhesive coating or in or on the first substrate, or any combination thereof:
      (a) a monomer, and optionally at least one comonomer;
      (b) an initiator, and optionally at least one co-initiator;
      (c) at least one crosslinking agent; and
      (d) at least one active agent deactivating agent.
2. The transdermal delivery device disposal system of item 1, further comprising a second substrate which is capable of being substantially adhered to the adhesive coating on the first substrate.
3. The transdermal delivery device disposal system of item 2, wherein an adhesive coating is disposed on one face of the second substrate.
4. The transdermal delivery device disposal system of item 3, further comprising one or more of the following components present at least in or on the adhesive coating on the second substrate or in or on the second substrate, or any combination thereof:
   (a) a monomer, and optionally at least one comonomer;
   (b) an initiator, an optionally at least one co-initiator;
   (c) at least one crosslinking agent; and
   (d) at least one active agent deactivating agent.
5. The transdermal delivery device disposal system item 3, wherein at least one of the first substrate and the second substrate are rigid.
6. The transdermal delivery device disposal system of item 3, wherein both the first substrate and the second substrate are rigid.
7. The transdermal delivery device disposal system of item 5, wherein at least one of the first substrate and the second substrate are non-planar.
8. The transdermal delivery device disposal system of item 6, wherein both the first substrate and the second substrate are non-planar.
9. The transdermal delivery device disposal system of item 7, wherein at least one of the first substrate and the second substrate define a recess configured and dimensioned to accept a transdermal delivery device therein.
10. The transdermal delivery device disposal system of item 1, wherein the first substrate exhibits one or more of the following properties:
   (a) substantially solvent impermeable;
   (b) substantially not solvent swellable;
   (c) tear resistant; and
   (d) cut resistant.
11. The transdermal delivery device disposal system of item wherein the component(s) present includes a monomer.
12. The transdermal delivery device disposal system of item 11, wherein the component(s) present includes at least one comonomer.
13. The transdermal delivery,device disposal system of item 11, wherein the components present further include an initiator.
14. The transdermal delivery device disposal system of item 11, wherein the components present further include at least one crosslinking agent.
15. The transdermal delivery device disposal system of item 14, wherein the components present further include at least one active agent deactivating agent.
16. The transdermal delivery device disposal system of item 15, wherein the active agent deactivating agent comprises an µ-opioid antagonist.
17. The transdermal delivery device disposal system of item 1, further comprising at least one adhesive backing material releasably disposed on the adhesive coating on the first substrate, such that the adhesive backing material can easily be removed from the adhesive coating.
18. A kit comprising:
   a transdermal delivery device comprising at least one active agent; and
   a transdermal delivery device disposal system, which comprises:
      (i) at least a first substrate having an adhesive coating on one face thereof; and
      (ii) one or more of the following components present at least in or on the adhesive coating or in or on the first substrate, or any combination thereof:
         (a) a monomer, and optionally at least one comonomer;
         (b) an initiator, and optionally at least one co-initiator,
         (c) at least one crosslinking agent; and
         (d) at least one active agent deactivating agent.
19. The kit item 18, further comprising a box, wherein the transdermal delivery device is disposed inside the box; and the transdermal delivery device disposal system is releasably attached to an outside portion of the box.
20. The kit of item 19, wherein one edge of the first substrate and one edge of a second substrate are bound together to form a booklet.
21. The kit of item 20, wherein each of the first substrate and the second substrate are rigid and non-planar.
22. The kit of item 21, wherein at least one of the first substrate and the second substrate defines a recess for receiving a transdermal delivery device therein.
23. The kit of item 20, wherein the transdermal delivery device, the first substrate, and the second substrate each have one face which is about the same size as one face of the others.
24. A kit comprising:
   a transdermal delivery device containing at least one active agent; and
   at least a first rigid, non-planar substrate having an adhesive coating on at least one face.
25. The kit of item 24, further comprising a second rigid non-planar substrate having an adhesive coating on at least one face thereof.
26. The kit of item 24, wherein the first substrate and the second substrate each define a recess configured and dimensional to receive a transdermal delivery device therein; and wherein the adhesive coatings on the first and second substrates are disposed at least on the portions of the first and second substrates which define the recesses.
27. The kit of item 24, further comprising one or more of the following components present at least in or on the first substrate, a second substrate, an adhesive coating on at least one face of the first substrate, an adhesive coating on at least one face of the second substrate, or any combination thereof:
   (a) a monomer, and optionally at least one comonomer;
   (b) an initiator, and optionally at least one co-initiator;
   (c) at least one crosslinking agent; and
   (d) at least one active agent deactivating agent.
28. A kit comprising:
   a transdermal delivery device comprising buprenorphine or any pharmaceutically acceptable form or derivative of buprenorphine; and
   at least a first substrate having an adhesive coating on one face thereof.
29. A kit comprising:
   a transdermal delivery device comprising fentanyl or any pharmaceutically acceptable form or derivative of fentanyl; and
   at least a first substrate having an adhesive coating on one face thereof.
30. A kit comprising:
   a transdermal delivery device comprising oxycodone or any pharmaceutically acceptable form or derivative of oxycodone; and
   at least a first substrate having an adhesive coating on one face thereof.
31. A transdermal delivery device disposal system comprising at least a first substrate having an adhesive coating on one face thereof, wherein the adhesive is a silicone-based adhesive.
32. A transdermal delivery device disposal system comprising at least a first substrate having an adhesive coating on one face thereof, wherein the adhesive is an acrylate-based adhesive.
33. A method of disposing of a transdermal delivery device comprising:
   adhering a transdermal delivery device to the adhesive coating on the first substrate of the transdermal delivery device disposal system of item 1.
34. A method of disposing of a transdermal delivery device by using a transdermal delivery device disposal system, the method comprising:
   adhering a transdermal delivery device to an adhesive coating on one face of a first substrate and having one or more of the following components present at least in or on the adhesive coating of the first substrate or in or on the first substrate, or any combination thereof:
      (a) a monomer, and optionally at least one comonomer;
      (b) an initiator, and optionally at least one co-initiator;
      (c) at least one crosslinking agent; and
      (d) at least one active agent deactivating agent;
   adhering the transdermal delivery device to an adhesive coating disposed on one face of a second substrate, the second substrate being capable of substantially adhering to the adhesive coating on the first substrate,
   wherein the transdermal delivery device disposal system comprises one or more of the following components present at least in or on the adhesive coating of the second substrate or in or on the second substrate, or any combination thereof:
      (a) a monomer, and optionally at least one comonomer;
      (b) an initiator, an optionally at least one co-initiator;
      (c) at least one crosslinking agent; and
      (d) at least one active agent deactivating agent.
35. A method for disposing of a transdermal delivery device comprising:
   providing a first rigid, non-planar substrate having an adhesive coating on at least one face thereof;
   providing a second rigid, non-planar substrate having an adhesive coating on at least one face thereof;
   wherein the first substrate and the second substrate each define a recess configured and dimensioned to receive a transdermal delivery device therein; wherein the adhesive coatings on the first and second substrates are disposed at least on portions of the first and second substrates which define the recesses;
   placing a transdermal delivery device substantially within the recess of the first substrate; and
   placing the second substrate over the first substrate and substantially aligning the recess of the second substrate over the transdermal delivery device; and
   adhering the first substrate to the second substrate to encapsulate, immobilize, and/or isolate the transdermal delivery device.
36. A transdermal delivery device disposal system comprising:
   at least a first substrate having an adhesive coating on one face thereof; and
   one or more of the following components present at least in or on the adhesive coating or in or on the first substrate, or any combination thereof:
      (a) a monomer, and optionally at least one comonomer;
      (b) an initiator, and optionally at least one co-initiator;
      (c) at least one crosslinking agent; and
      (d) at least one non-µ-opioid deactivating agent.
37. The transdermal delivery device disposal system of item 36, further comprising a second substrate which is capable of being substantially adhered to the adhesive coating on the first substrate.
38. The transdermal delivery device disposal system of item 37, wherein an adhesive coating is disposed on one face of the second substrate.
39. The transdermal delivery device disposal system of item 38, further comprising one or more of the following components present at least in or on the adhesive coating of the second substrate or in or on the second substrate, or any combination thereof:
   (a) a monomer, and optionally at least one comonomer;
   (b) an initiator, an optionally at least one co-initiator;
   (c) at least one crosslinking agent; and
   (d) at least one non-µ-opioid deactivating agent.
40. The transdermal delivery device disposal system of item 38, wherein at least one of the first substrate or the second substrate are rigid.
41. The transdermal delivery device disposal system of item 40, wherein both the first substrate and the second substrate are rigid.
42. The transdermal delivery device disposal system of item 40, wherein at least one of the first substrate and the second substrate are non-planar.
43. The transdermal delivery device disposal system of item 41, wherein both the first substrate and the second substrate are non-planar.
44. The transdermal delivery device disposal system of item 42, wherein at least one of the first substrate or the second substrate define a recess configured and dimensioned to accept a transdermal delivery device therein.
45. The transdermal delivery device disposal system of item 36, wherein the first substrate exhibits one or more of the following properties:
   (a) substantially solvent impermeable;
   (b) substantially not solvent swellable;
   (c) tear resistant; and
   (d) cut resistant.
46. The transdermal delivery device disposal system of item 36, wherein the component(s) present includes a monomer.
47. The transdermal delivery device disposal system of item 46, wherein the component(s) present includes at least one comonomer.
48. The transdermal delivery device disposal system of item 46, wherein the components present further include an initiator.
49. The transdermal delivery device disposal system of item 46, wherein the components present further include at least one crosslinking agent.
50. The transdermal delivery device disposal system of item 49, wherein the components present further include at least one non-µ-opioid deactivating agent.

## Claims

1. Use of an article comprising:
at least a first substrate having an adhesive coating on one face thereof; and
one or more of the following components present at least in or on the adhesive coating or in or on the first substrate, or any combination thereof:
(a) a monomer, and optionally at least one comonomer;
(b) an initiator, and optionally at least one co-initiator;
(c) at least one crosslinking agent; and
(d) at least one active agent deactivating agent.
as a disposal system for a transdermal delivery device

2. The use of claim 1, wherein said first substrate is flexible.

3. The use of claim 1, further comprising a second substrate which is capable of being substantially adhered to the adhesive coating on the first substrate.

4. The use of claim 3 wherein an adhesive coating is disposed on one face of the second substrate.

5. The use of claim 4, further comprising one or more of the following components present at least in or on the adhesive coating on the second substrate or in or on the second substrate, or any combination thereof:
(a) a monomer, and optionally at least one comonomer;
(b) an initiator, an optionally at least one co-initiator;
(c) at least one crosslinking agent; and
(d) at least one active agent deactivating agent.

6. The use of claim 3, wherein at least one of the first substrate and the second substrate are flexible.

7. The use of claim 1, wherein the first substrate exhibits one or more of the following properties:
(a) substantially solvent impermeable;
(b) substantially not solvent swellable;
(c) tear resistant; and
(d) cut resistant.

8. The use of claim 1, wherein the component(s) present includes a monomer.

9. The use of claim 8, wherein the component(s) present includes at least one comonomer.

10. The use of claim 8, wherein the components present further include an initiator.

11. The use of claim 8, wherein the components present further include at least one crosslinking agent.

12. The use of claim 11, wherein the components present further include at least one active agent deactivating agent.

13. The use of claim 12, wherein the active agent deactivating agent comprises an µ-opioid antagonist.

14. The use of claim 1, wherein at least one adhesive backing material is releasably disposed on the adhesive coating on the first substrate, such that the adhesive backing material can easily be removed from the adhesive coating.

15. A kit comprising:
a transdermal delivery device comprising buprenorphine or any pharmaceutically acceptable form or derivative of buprenorphin or fentanyl or any pharmaceutically acceptable form or derivative of fentanyl; and
a disposal system for the transdermal delivery device comprising at least a first substrate of the disposal system having an adhesive coating on one face thereof.
